# EUROPÄISCHE PATENTSCHRIFT

(11) **EP 1 049 695 B1**
(45) Veröffentlichungstag und Bekanntmachung des Hinweises auf die Patenterteilung: **13.02.2002**
(21) Anmeldenummer: 98959821.4
(22) Anmeldetag: 31.10.1998
(51) Int. Cl.: C07D 487/04, A61K 31/53

(54) **2-PHENYL-SUBSTITUIERTE IMIDAZOTRIAZINONE ALS PHOSPHODIESTERASE INHIBITOREN**
2-PHENYL SUBSTITUTED IMIDAZOTRIAZINONES AS PHOSPHODIESTERASE INHIBITORS
IMIDAZOTRIAZINONES A SUBSTITUTION 2-PHENYLE UTILISEES COMME INHIBITEURS DES PHOSPHODIESTERASES

(30) Priorität: 12.11.1997 DE 19750085; 23.03.1998 DE 19812462; 04.09.1998 DE 19840289
(43) Veröffentlichungstag der Anmeldung: 08.11.2000
(60) Teilanmeldung: 01123321.0
(73) Patentinhaber: BAYER AG, 51368 Leverkusen (DE)
(72) Erfinder: NIEWÖHNER, Ulrich, D-42929 Wermelskirchen (DE); ES-SAYED, Mazen, D-42115 Wuppertal (DE); HANING, Helmut, D-42115 Wuppertal (DE); SCHENKE, Thomas, D-51469 Bergisch Gladbach (DE); SCHLEMMER, Karl-Heinz, D-42113 Wuppertal (DE); KELDENICH, Jörg, D-42113 Wuppertal (DE); BISCHOFF, Erwin, D-42115 Wuppertal (DE); PERZBORN, Elisabeth, D-42327 Wuppertal (DE); DEMBOWSKY, Klaus, D-69198 Schriesheim (DE); SERNO, Peter, D-51467 Bergisch Gladbach (DE); NOWAKOWSKI, Marc, D-42115 Wuppertal (DE)
(86) Internationale Anmeldenummer: EP9806910
(87) Internationale Veröffentlichungsnummer: WO9924433

(56) Entgegenhaltungen:
- EP-A- 0 463 756
- EP-A- 0 812 845
- WO-A-93/06104
- WO-A-93/07149
- WO-A-93/12095
- WO-A-94/00453
- WO-A-94/05661
- WO-A-94/28902
- WO-A-96/16657
- DE-A- 2 255 172
- DE-A- 2 364 076
- DE-A- 2 811 780
- CHARLES I ET AL: "BICYCLIC HETEROCYCLES WITH NITROGEN AT THE RING JUNCTION. PART 2.1 APPLICATION OF THE DAKIN-WEST REACTION TO THE SYNTHESIS OF IMIDAZO - 5,1-F-1,2,4-TRIAZIN-4(3H)-ONES" JOURNAL OF THE CHEMICAL SOCIETY, PERKIN TRANSACTIONS 1, Nr. 5, Mai 1980, Seiten 1139-1146, XP002027191
- J.MED.CHEM. Bd. 39, 1996, WASHINGTON, Seiten 1635 - -1644
- NEW ENGL.J.MED. Bd. 326, 1992, Seite 90
- BIOCHEM.PHARMACOL. Bd. 50, 1995, Seite 1577
- DRUG NEWS & PERSEPCT. Bd. 6, 1993, Seite 150
- DRABER W., FUJITA T.: 'Rational Approaches to Structure,Activity and Ecotoxicology of Agrochemicals', 1992, CRC PRESS, BOCA RATON
- KOROLKOVACS A.: 'Essentials of Medicinal Chemistry', 1988, WILEY INTERSCIENCE

## Beschreibung

Die vorliegende Erfindung betrifft 2-Phenyl-substituierte Imidazotriazinone, Verfahren zu ihrer Herstellung und ihre Verwendung als Arzneimittel, insbesondere als Inhibitoren cGMP-metabolisierender Phosphodiesterasen.

In der Offenlegungsschrift DE 28 11 780 sind Imidazotriazine als Bronchodilatoren mit spasmolytischer Aktivität und Hemmaktivität gegen cyclisches Adenosinmonophosphat metabolisierende Phosphodiesterasen (cAMP-PDE's, Nomenklatur nach Beavo: PDE-III und PDE-IV) beschrieben. Eine Hemmwirkung gegen cyclisches Guanosin-monophosphat metabolisierende Phosphodiesterasen (cGMP-PDE's, Nomenklatur nach Beavo und Reifsnyder (Trends in Pharmacol. Sci. 11, 150-155, 1990) PDE-I, PDE-II und PDE-V) ist nicht beschrieben. Es werden keine Verbindungen beansprucht, die eine Sulfonamidgruppe im Arylrest in der 2-Position enthalten. Weiterhin werden Imidazotriazinone in FR 22 13 058, CH 59 46 71, DE 22 55 172, DE 23 64 076 und EP 000 9384 beschrieben, die in der 2-Position keinen substituierten Arylrest besitzen, und ebenfalls als Bronchodilatatoren mit cAMP-PDE inhibitorischer Wirkung beschrieben werden.

In WO 94/28902 werden Pyrazolopyrimidinone beschrieben, die sich für die Behandlung von Impotenz eignen.

Die erfindungsgemäßen Verbindungen sind potente Inhibitoren von entweder einer oder mehrerer der cyclisches Guanosin 3',5'-monophosphat metabolisierenden Phosphodiesterasen (cGMP-PDE's). Entsprechend der Nomenklatur von Beavo und Reifsnyder (Trends in Pharmacol. Sci. 11, 150-155, 1990) handelt es sich um die Phosphodiesterase Isoenzyme PDE-I, PDE-II und PDE-V.

Ein Anstieg der cGMP-Konzentration kann zu heilsamen, antiaggregatorischen, antithrombotischen, antiproliferativen, antivasospastischen, vasodilatierenden, natriuretischen und diuretischen Effekten führen. Es kann die Kurz- oder Langzeitmodulation der vaskulären und kardialen Inotropie, den Herzrhythmus und die kardiale Erregungsleitung beeinflussen (J.C. Stoclet, T. Keravis, N. Komas and C. Kugnier, Exp. Opin. Invest. Drugs (1995), 4 (11), 1081-1100).

Die vorliegende Erfindung betrifft die in der Tabelle A aufgeführten Verbindungen

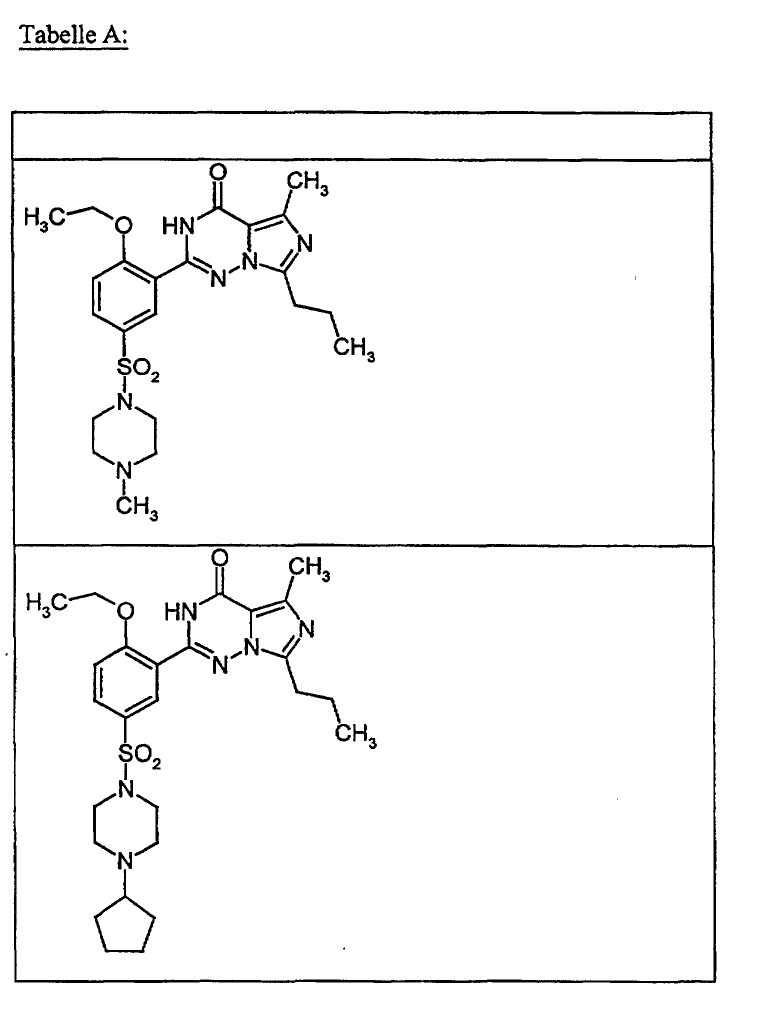

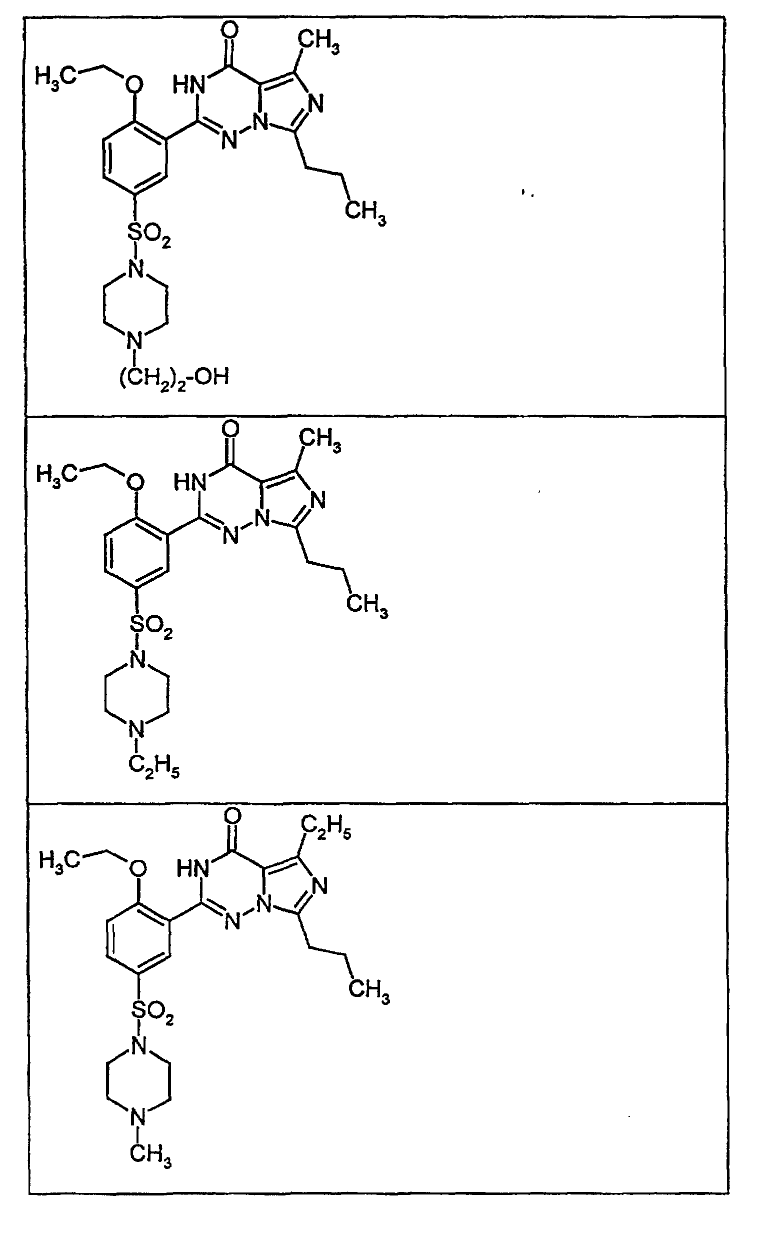

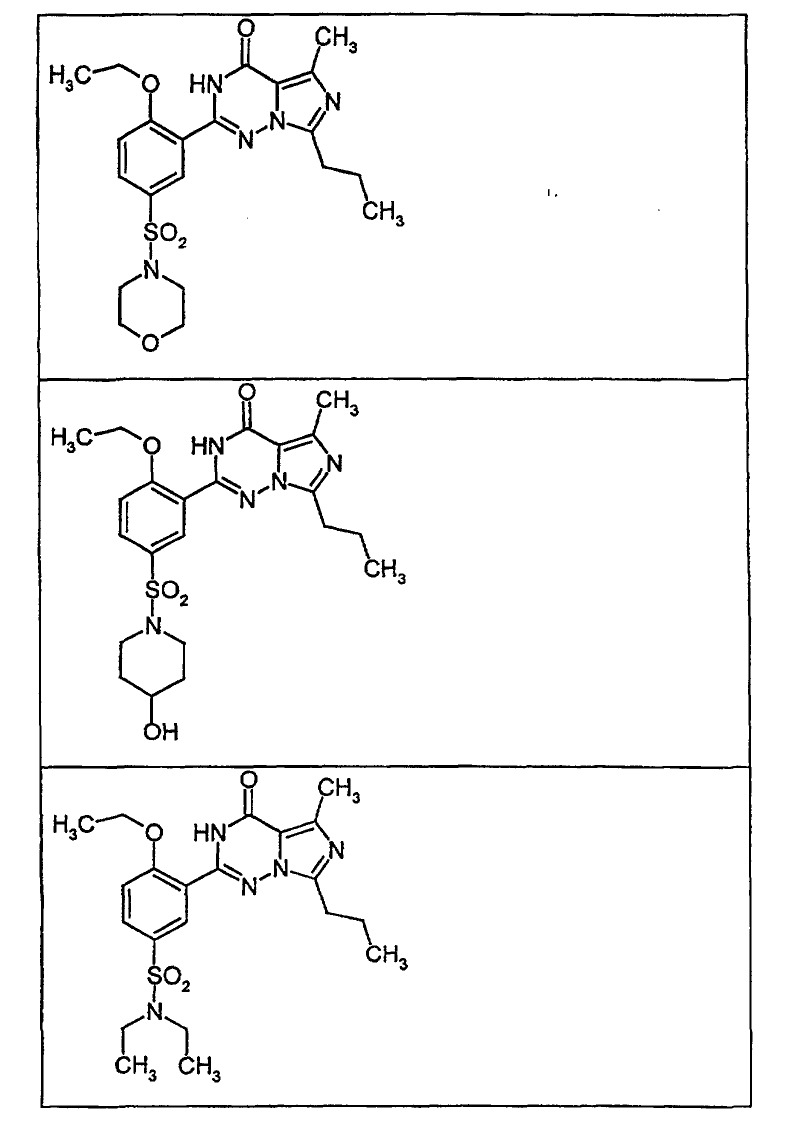

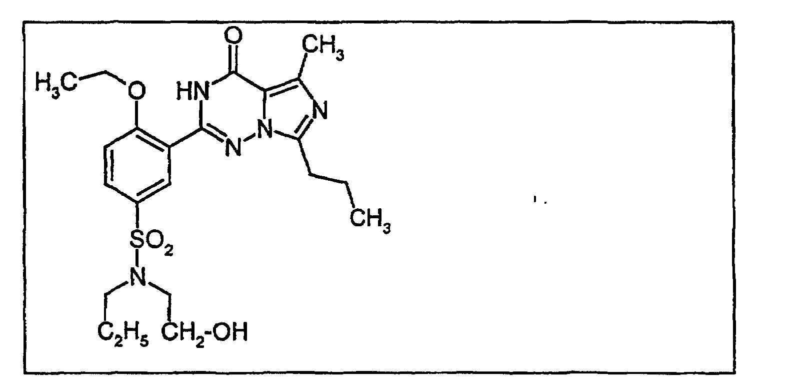

Sowie deren Salze. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

. Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen, insbesondere die Salze, können auch als Hydrate vorliegen. Im Rahmen der Erfindung werden unter Hydraten solche Verbindungen verstanden, die im Kristall Wasser enthalten. Solche Verbindungen können ein oder mehrere, typischerweise 1 bis 5, Äquivalente Wasser enthalten. Hydrate lassen sich beispielsweise herstellen, indem man die betreffende Verbindung aus Wasser oder einem wasserhaltigen Lösungsmittel kristallisiert.

Erfindungsgemäße Salze bzw. Hydrate sind beispielsweise folgende Verbindungen:

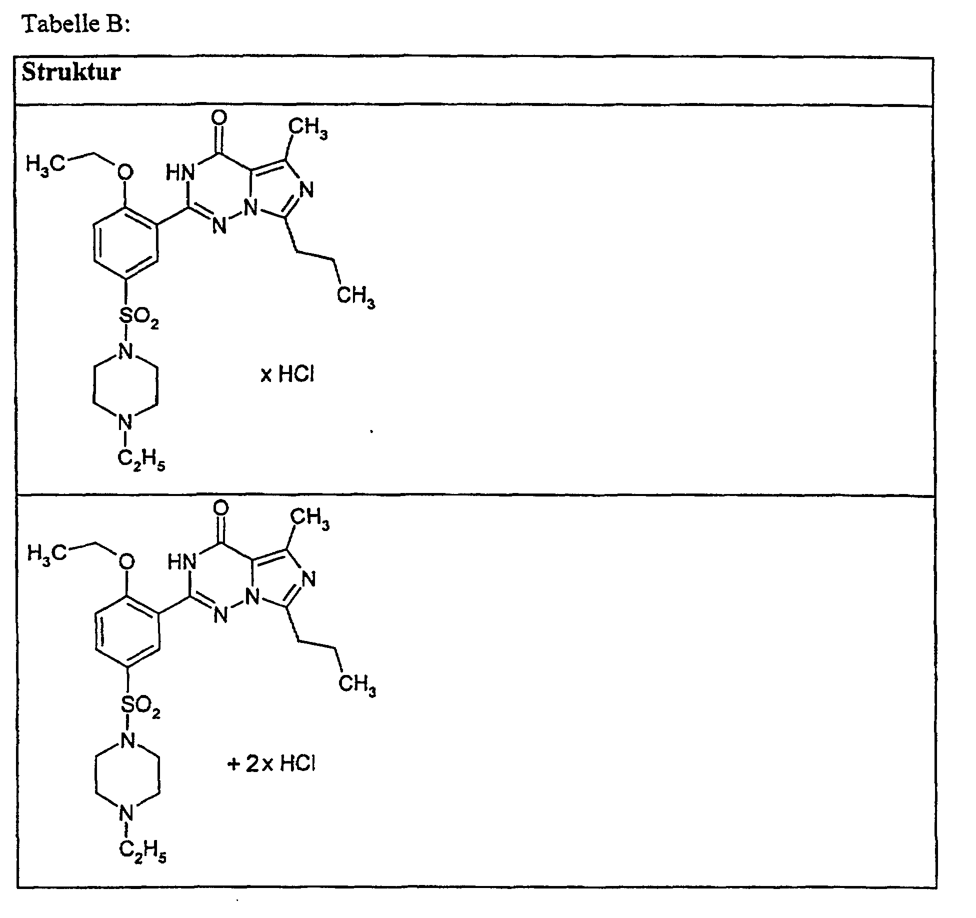

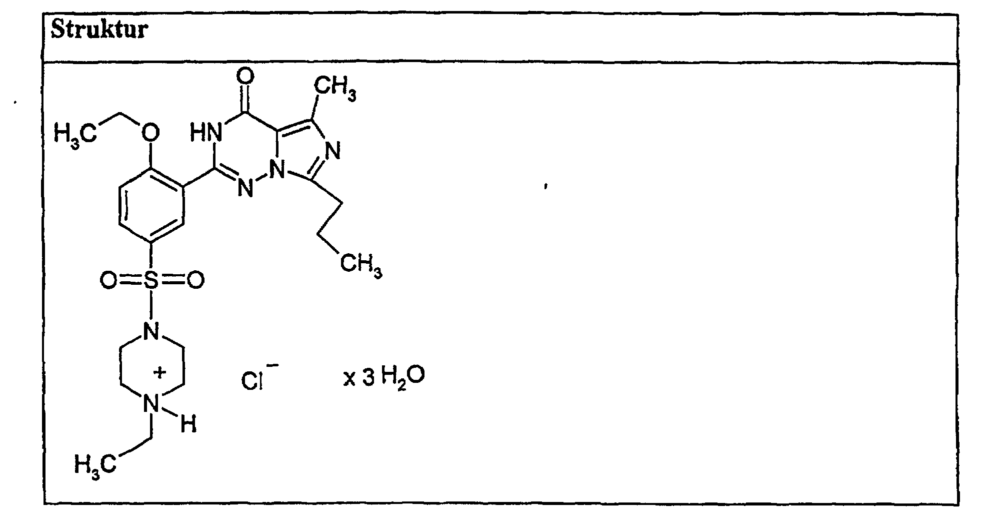

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Außerdem wurde ein Verfahren zur Herstellung der erfindungsgemäßen Verbindungen gefunden, dadurch gekennzeichnet, daß man

zunächst Verbindungen der allgemeinen Formel (II) 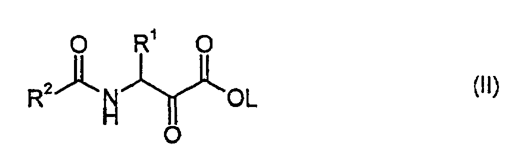 in welcher
R¹ für Methyl oder Ethyl und R² für Propyl steht
und
- L: für geradkettiges oder verzweigtes Alkyl mit bis zu 4 Kohlenstoffatomen steht,
mit Verbindungen der allgemeinen Formel (III) 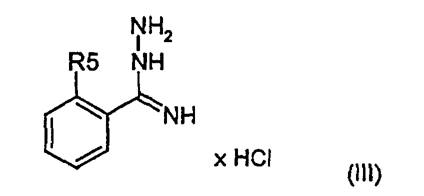 in der R⁵ für einen Ethoxyrest steht,
in einer Zweistufenreaktion in den Systemen Ethanol und Phosphoroxytrichlorid / Dichlorethan in die Verbindungen der allgemeinen Formel (IV) 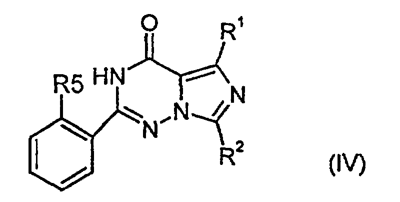 in welcher
R¹, R² und R⁵ die oben angegebene Bedeutung haben,
überführt, in einem weiteren Schritt mit Chlorsulfonsäure zu den Verbindungen der allgemeinen Formel (V) 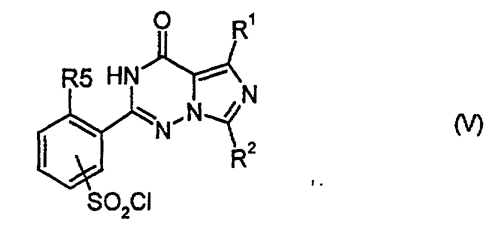 in welcher
R¹, R² und R⁵, die oben angegebene Bedeutung haben,
und abschließend mit den entsprechenden Aminen in inerten Lösemitteln umsetzt.

Das erfindungsgemäße Verfahren kann durch folgendes Formelschema beispielhaft erläutert werden:

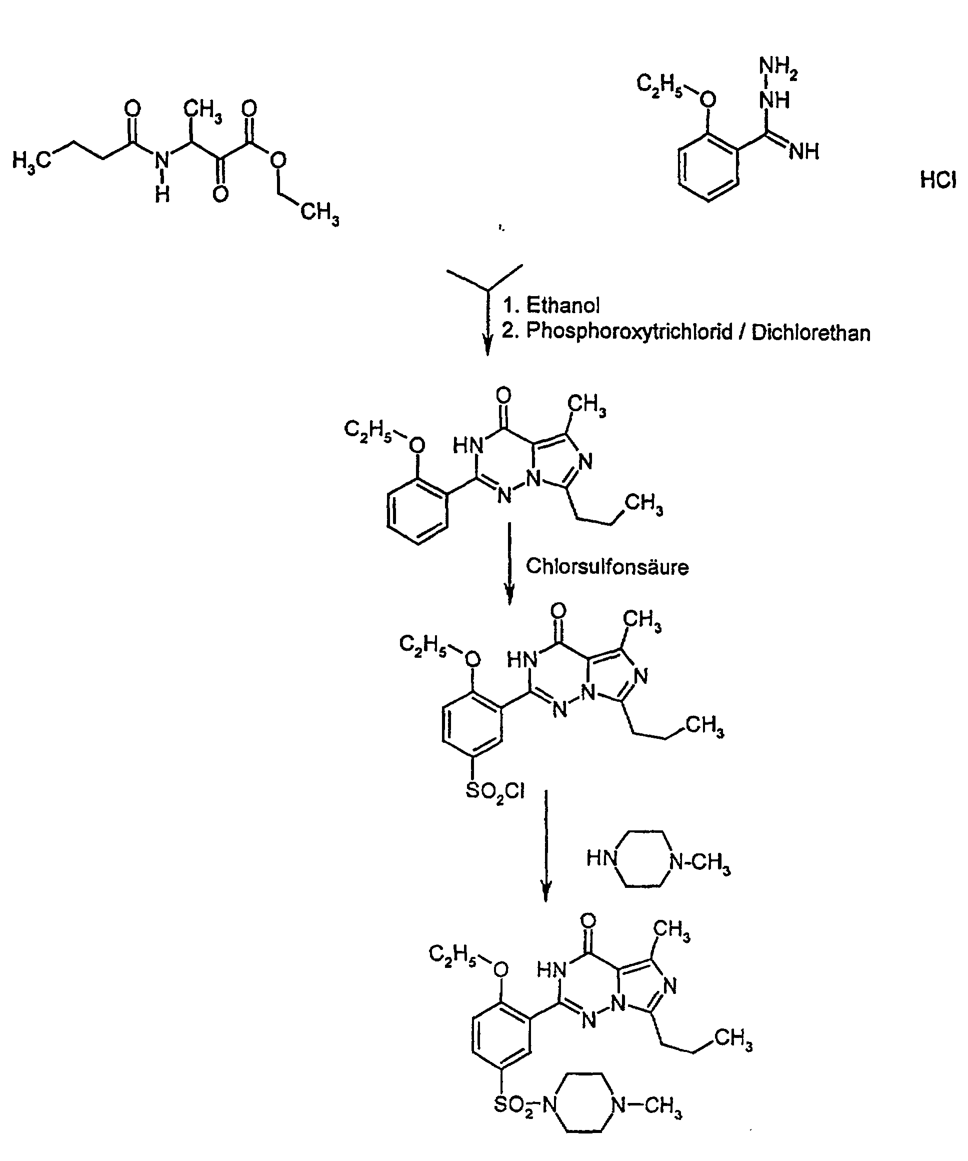

Als Lösemittel für die einzelnen Schritte eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden. Besonders bevorzugt ist für den ersten Schritt Ethanol und für den zweiten Schritt Dichlorethan.

Die Reaktionstemperatur kann im allgemeinen in einem größeren Bereich variiert werden. Im allgemeinen arbeitet man in einem Bereich von -20°C bis 200°C, bevorzugt von 0°C bis 70°C.

Die erfindungsgemäßen Verfahrensschritte werden im allgemeinen bei Normaldruck durchgeführt. Es ist aber auch möglich, sie bei Überdruck oder bei Unterdruck durchzuführen (z.B. in einem Bereich von 0,5 bis 5 bar).

Die Umsetzung zu den Verbindungen der allgemeinen Formel (V) erfolgt in einem Temperaturbereich von 0°C bis Raumtemperatur und Normaldruck.

Die Umsetzung mit den entsprechenden Aminen erfolgt in einem der oben aufgeführten chlorierten Kohlenwasserstoffe, vorzugsweise in Dichlormethan.

Die Reaktionstemperatur kann im allgemeinen in einem größeren Bereich variiert werden. Im allgemeinen arbeitet man in einem Bereich von -20°C bis 200°C, bevorzugt von 0°C bis Raumtemperatur.

Die Umsetzung wird im allgemeinen bei Normaldruck durchgeführt. Es ist aber auch möglich, sie bei Überdruck oder bei Unterdruck durchzuführen (z.B. in einem Bereich von 0,5 bis 5 bar).

Die Verbindungen der allgemeinen Formel (II) sind teilweise bekannt oder neu und können dann hergestellt werden, indem man

Verbindungen der allgemeinen Formel (VII)

R²-CO-T (VII)

in welcher
- R²: die oben angegebene Bedeutung hat
und
- T: für Halogen, vorzugsweise für Chlor steht,
zunächst durch Umsetzung mit Verbindungen der allgemeinen Formel (VIII) 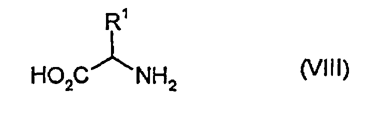 in welcher
- R¹: die oben angegebene Bedeutung hat
in inerten Lösemitteln, gegebenenfalls in Anwesenheit einer Base und Trimethylsilylchlorid in die Verbindungen der allgemeinen Formel (IX) 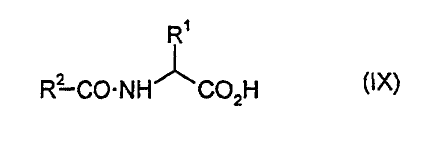 in welcher
R¹ and R² die oben angegebene Bedeutung haben,
überführt und abschließend mit der Verbindung der Formel (X) 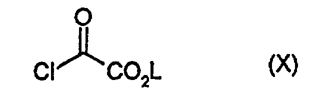 worin L die oben angegebene Bedeutung hat,
in inerten Lösemitteln, gegebenenfalls in Anwesenheit einer Base umsetzt.

Als Lösemittel für die einzelnen Schritte des Verfahrens eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden. Besonders bevorzugt ist für den ersten Schritt Dichlormethan und für den zweiten Schritt ein Gemisch aus Tetrahydrofuran und Pyridin.

Als Basen eignen sich im allgemeinen Alkalihydride oder -alkoholate, wie beispielsweise Natriumhydrid oder Kalium-tert.butylat, oder cyclische Amine, wie beispielsweise Piperidin, Pyridin, Dimethylaminopyridin oder C₁-C₄-Alkylamine, wie beispielsweise Triethylamin. Bevorzugt sind Triethylamin, Pyridin und/oder Dimethylaminopyridin.

Die Base wird im allgemeinen in einer Menge von 1 mol bis 4 mol, bevorzugt von 1,2 mol bis 3 mol jeweils bezogen auf 1 mol der Verbindung der Formel (X) eingesetzt.

Die Reaktionstemperatur kann im allgemeinen in einem größeren Bereich variiert werden. Im allgemeinen arbeitet man in einem Bereich von -20°C bis 200°C, bevorzugt von 0°C bis 100°C.

Die Verbindungen der allgemeinen Formeln (VII), (VIII), (IX) und (X) sind an sich bekannt oder nach üblichen Methoden herstellbar.

Die Verbindungen der allgemeinen Formel (III) können hergestellt werden, indem man

Verbindungen der allgemeinen Formel (XI) 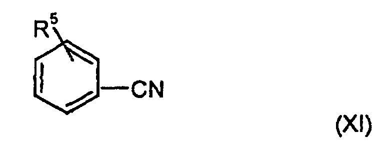 in welcher
R⁵ die oben angegebene Bedeutung hat,
mit Ammoniumchlorid in Toluol und in Anwesenheit von Trimethylaluminium in Hexan in einem Temperaturbereich von -20°C bis Raumtemperatur, vorzugsweise bei 0°C und Normaldruck umsetzt und das entstehende Amidin, gegebenenfalls in situ, mit Hydrazin-hydrat umsetzt.

Die Verbindungen der allgemeinen Formel (XI) sind an sich bekannt oder nach üblichen Methoden herstellbar.

Die Verbindungen der allgemeinen Formel (IV) sind teilweise bekannt oder neu und können dann nach bekannten Methoden [vgl. David R. Marshall, Chemistry and Industry, 2 May 1983, 331-335] hergestellt werden.

Die Verbindungen der allgemeinen Formel (V) sind an sich neu, können aber aus den Verbindungen der allgemeinen Formel (IV) nach der Publikation Organikum, VEB Deutscher Verlag der Wissenschaften, Berlin 1974, Seite 338 - 339, hergestellt werden.

Die erfindungsgemäßen Verbindungen zeigen ein nicht vorhersehbares, wertvolles pharmakologisches Wirkspektrum.

Sie inhibieren entweder eine oder mehrere der c-GMP metabolisierenden Phosphodiesterasen (PDE I, PDE II und PDE V). Dies führt zu einem Anstieg von c-GMP. Die differenzierte Expression der Phosphodiesterasen in verschiedenen Zellen, Geweben und Organen, ebenso wie die differenzierte subzelluläre Lokalisation dieser Enzyme, ermöglichen in Verbindung mit den erfindungsgemäßen selektiven Inhibitoren, eine selektive Adressierung der verschiedenen von cGMP regulierten Vorgänge.

Außerdem verstärken die erfindungsgemäßen Verbindungen die Wirkung von Substanzen, wie beispielsweise EDRF (Endothelium derived relaxing factor), ANP (atrial natriuretic peptide), von Nitrovasodilatoren und allen anderen Substanzen, die auf eine andere Art als Phosphodiesterase-Inhibitoren die cGMP-Konzentration erhöhen.

Sie können daher in Arzneimitteln zur Behandlung von cardiovaskulären Erkrankungen wie beispielsweise zur Behandlung des Bluthochdrucks, neuronaler Hypertonie, stabiler und instabiler Angina, peripheren und kardialen Gefäßerkrankungen, von Arrhythmien, zur Behandlung von thromboembolischen Erkrankungen und Ischämien wie Myokardinfarkt, Hirnschlag, transistorischen und ischämischen Attacken, Angina pectoris, periphere Durchblutungsstörungen, Verhinderung von Restenosen nach Thrombolysetherapie, percutaner transluminaler Angioplastie (PTA), percutan transluminalen Koronarangioplastien (PTCA) und Bypass eingesetzt werden. Weiterhin können sie auch Bedeutung für cerebrovaskuläre Erkrankungen haben. Die relaxierende Wirkung auf glatte Muskulatur macht sie geeignet für die Behandlung von Erkrankungen des Urogenitalsystems wie Prostatahypertrophie, Inkontinenz sowie insbesondere zur Behandlung der erektilen Dysfunktion und der weiblichen sexuellen Dysfünktion.

### Aktivität der Phosphordiesterasen (PDE's)

Die c-GMP stimulierbare PDE II, die c-GMP hemmbare PDE III und die cAMP spezifische PDE IV wurden entweder aus Schweine- oder Rinderherzmyokard isoliert. Die Ca²⁺-Calmodulin stimulierbare PDE I wurde aus Schweineaorta, Schweinehirn oder bevorzugt aus Rinderaorta isoliert. Die c-GMP spezifische PDE V wurde aus Schweinedünndarm, Schweineaorta, humanen Blutplättchen und bevorzugt aus Rinderaorta gewonnen. Die Reinigung erfolgte durch Anionenaustauschchromatographie an MonoQ^{R} Pharmacia im wesentlichen nach der Methode von M. Hoey and Miles D. Houslay, Biochemical Pharmacology, Vol. 40, 193-202 (1990) und C. Lugman et al. Biochemical Pharmacology Vol. 35 1743-1751(1986).

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq ³HcAMP oder ³HcGMP enthält. Die Endkonzentration der entsprechenden Nucleotide ist 10⁻⁶ mol/l. Die Reaktion wird durch Zugabe des Enzyms gestartet, die Enzymmenge ist so bemessen, daß während der Inkubationszeit von 30 min ca 50% des Substrates umgesetzt werden. Um die cGMP stimulierbare PDE II zu testen, wird als Substrat ³HcAMP verwendet und dem Ansatz 10⁻⁶ mol/l nicht markiertes cGMP zugesetzt. Um die Ca²⁺-Calmodulinabhängige PDE I zu testen, werden dem Reaktionsansatz noch CaCl₂ 1 µM und Calmodulin 0,1 µM zugesetzt. Die Reaktion wird durch Zugabe von 100 µl Acetonitril, das 1 mM cAMP und 1 mM AMP enthält, gestoppt. 100 µl des Reaktionsansatzes werden auf der HPLC getrennt und die Spaltprodukte "Online" mit einem Durchflußscintillationszähler quantitativ bestimmt. Es wird die Substanzkonzentration gemessen, bei der die Reaktionsgeschwindigkeit um 50% vermindert ist. Zusätzlich wurde zur Testung der "Phosphodiesterase [³H] cAMP-SPA enzyme assay" und der "Phosphodiesterase [³H] cGMP-SPA enzyme assay" der Firma Amersham Life Science verwendet. Der Test wurde nach dem vom Hersteller angegebenen Versuchsprotokoll durchgeführt. Für die Aktivitätsbestimmung der PDEII wurde der [³H] cAMP SPA assay verwendet, wobei dem Reaktionsansatz 10⁻⁶ M cGMP zur Aktivierung des Enzyms zugegeben wurde. Für die Messung der PDEI wurden Calmodulin 10⁻⁷ M und CaCl₂ 1µM zum Reaktionsansatz zugegeben. Die PDEV wurde mit dem [³H] cGMP SPA assay gemessen.

### Inhibition der Phosphodiesterasen in vitro

**Tabelle 1:**

| **Bsp.-Nr.** | **PDE I** | **PDE II** | **PDE V** |
|---|---|---|---|
| | **IC**_{**50**} **[nM]** | **IC**_{**50**} **[nM]** | |
| 1 | 300 | >1000 | 2 |
| 2 | 200 | >1000 | 2 |
| 3 | 100 | >1000 | 1 |
| 4 | 200 | >1000 | 3 |
| 5 | 100 | >1000 | 4 |
| 6 | 800 | >1000 | 4 |
| 8 | 300 | >1000 | 10 |
| 9 | 50 | >1000 | 3 |
| | | | |

### Herstellungsbeispiele

Grundsätzlich führt die Inhibition einer oder mehrerer Phosphodiesterasen dieses Typs zu einer Erhöhung der cGMP-Konzentration. Dadurch sind die Verbindungen interessant für alle Therapien, in denen eine Erhöhung der cGMP-Konzentration als heilsam angenommen werden kann.

Die Untersuchung der cardiovaskulären Wirkungen wurden an SH-Ratten und Hunden durchgeführt. Die Substanzen wurden intravenös oder oral appliziert.

Die Untersuchung auf erektionsauslösende Wirkung wurde am wachen Kaninchen durchgeführt [Naganuma H, Egashira T, Fuji J, Clinical and Experimental Pharmacology and Physiology 20, 177-183 (1993)]. Die Substanzen wurden intravenös, oral oder parenteral appliziert.

Die neuen Wirkstoffe sowie ihre physiologisch unbedenklichen Salze (z.Bsp. Hydrochloride, Maleinate oder Lactate) können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, z.Bsp.perlingual, buccal, intravenös, nasal, rektal oder inhalativ.

Für die Anwendung beim Menschen werden bei oraler Administration Dosierungen von 0,001 bis 50 mg/kg vorzugsweise 0,01 mg/kg - 20 mg/kg sinnvollerweise verabreicht. Bei parenteraler Administration, wie z.B. über Schleimhäute nasal, buccal, inhalativ, ist eine Dosierung von 0,001 mg/kg - 0,5 mg/kg sinnvoll.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der oben genannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Die erfindungsgemäßen Verbindungen sind auch zur Anwendung in der Tiermedizin geeignet. Für Anwendungen in der Tiermedizin können die Verbindungen oder ihre nicht toxischen Salze in einer geeigneten Formulierung in Übereinstimmung mit den allgemeinen tiermedizinischen Praxen verabreicht werden. Der Tierarzt kann die Art der Anwendung und die Dosierung nach Art des zu behandelnden Tieres festlegen.

### Ausgangsverbindungen

### Beispiel 1A

2-Butyrylaminopropionsäure 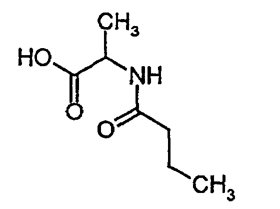 22,27 g (250 mmol) D,L-Alanin und 55,66g (550 mmol) Triethylamin werden in 250 ml Dichlormethan gelöst und die Lösung auf 0°C abgekühlt. 59,75 g (550 mmol) Trimethylsilylchlorid werden zugetropft und die Lösung 1 Stunde bei Raumtemperatur und eine Stunde bei 40°C gerührt. Nach dem Abkühlen auf -10°C werden 26,64 g (250 mmol) Buttersäurechlorid zugetropft und die resultierende Mischung 2 Stunden bei -10°C und eine Stunde bei Raumtemperatur gerührt.

Unter Eiskühlung werden 125 ml Wasser zugetropft und die Reaktionsmischung 15 Minuten bei Raumtemperatur gerührt. Die wäßrige Phase wird bis zur Trockene eingedampft, der Rückstand mit Aceton verrieben und die Mutterlauge abgesaugt. Nach dem Entfemen des Lösungsmittels wird der Rückstand chromatographiert . Das erhaltene Produkt wird in 3N Natronlauge gelöst und die resultierende Lösung bis zur Trockene eingedampft. Es wird mit konz. HCl aufgenommen und wieder bis zur Trockene eingedampft. Es wird mit Aceton verrührt, vom ausgefallenen Feststoff abgesaugt und das Lösungsmittel im Vakuum entfernt. Man erhält 28,2 g (71 %) eines zähen Öls, das nach einiger Zeit kristallisiert.

200 MHz ¹H-NMR (DMSO-d6): 0.84, t, 3H; 1.22, d, 3H; 1.50, hex, 2H; 2.07, t, 2H; 4.20, quin., 1H; 8.09, d, 1H.

### Beispiel 2A

2-Ethoxybenzonitril 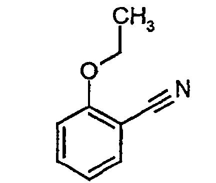 25 g (210 mmol) 2-Hydroxybenzonitril werden mit 87 g Kaliumcarbonat und 34,3 g (314,8 mmol) Ethylbromid in 500 ml Aceton über Nacht refluxiert. Es wird vom Feststoff abfiltriert, das Lösungsmittel im Vakuum entfernt und der Rückstand im Vakuum destilliert. Man erhält 30,0 g (97 %) einer farblosen Flüssigkeit.
200 MHz ¹H-NMR (DMSO-d6): 1.48, t, 3H; 4.15, quart., 2H; 6.99, dt, 2H; 7.51, dt, 2H.

### Beispiel 3A

2-Ethoxybenzamidinhydrochlorid 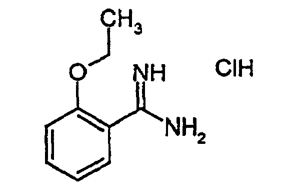 21,4 g (400 mmol) Ammoniumchlorid werden in 375 ml Toluol suspendiert und die Suspension auf 0°C abgekühlt. 200 ml einer 2M Lösung von Trimethylaluminium in Hexan werden zugetropft und die Mischung bis zur beendeten Gasentwicklung bei Raumtemperatur gerührt. Nach Zugabe von 29,44 g (200 mmol) 2-Ethoxybenzonitril wird die Reaktionsmischung über Nacht bei 80°C (Bad) gerührt.

Die abgekühlte Reaktionsmischung wird unter Eiskühlung zu einer Suspension aus 100 g Kieselgel und 950 ml Chloroform gegeben und die Mischung 30 Minuten bei Raumtemperatur gerührt. Es wird abgesaugt und mit der gleichen Menge Methanol nachgewaschen. Die Mutterlauge wird eingedampft, der erhaltene Rückstand mit einer Mischung aus Dichlormethan und Methanol (9:1) verrührt, der Feststoff abgesaugt und die Mutterlauge eingedampft. Man erhält 30,4 g (76 %) farblosen Feststoff.
200 MHz ¹H-NMR (DMSO-d6): 1.36, t, 3H; 4.12, quart., 2H; 7.10, t, 1H; 7.21, d, 1H; 7.52, m, 2H; 9.30, s, breit, 4H.

### Beispiel 4A

2-(2-Ethoxy-phenyl)-5-methyl-7-propyl-3*H*-imidazo[5,1-f][1,2,4]triazin-4-on 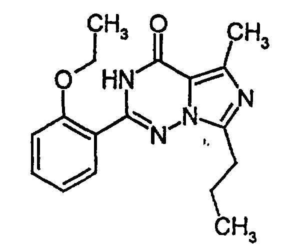 7,16 g (45 mmol) 2-Butyrylamino-propionsäure werden mit 10,67 g Pyridin in 45 ml THF gelöst und nach Zugabe einer Spatelspitze DMAP zum Rückfluß erhitzt. 12,29 g (90 mmol) Oxalsäure-ethylesterchlorid werden langsam zugetropft und die Reaktionsmischung wird 3 Stunden refluxiert. Es wird auf Eiswasser gegossen, dreimal mit Ethylacetat extrahiert, über Natriumsulfat getrocknet und einrotiert. Der Rückstand wird in 15 ml Ethanol aufgenommen und mit 2,15 g Natriumhydrogencarbonat 2,5 Stunden refluxiert. Die abgekühlte Lösung wird filtriert.

Zu einer Lösung von 9,03 g (45 mmol) 2-Ethoxybenzamidinhydrochlorid in 45 ml Ethanol tropft man unter Eiskühlung 2,25 g (45 mmol) Hydrazinhydrat zu und rührt die resultierende Suspension noch 10 Minuten bei Raumtemperatur. Zu dieser Reaktionsmischung gibt man die oben beschriebene ethanolische Lösung und rührt 4 Stunden bei 70°C Badtemperatur. Nach Filtration wird eingedampft, der Rückstand zwischen Dichlormethan und Wasser verteilt, die organische Phase über Natriumsulfat getrocknet und das Lösungsmittel im Vakuum entfernt.

Dieser Rückstand wird in 60 ml 1,2-Dichlorethan gelöst und nach Zugabe von 7,5 ml Phosphoroxychlorid 2 Stunden refluxiert. Es wird mit Dichlormethan verdünnt und durch Zugabe von Natriumhydrogencarbonatlösung und festem Natriumhydrogencarbonat neutralisiert. Die organische Phase wird getrocknet und das Lösungsmittel im Vakuum entfernt. Chromatographie mit Ethylacetat und Kristallisation ergeben 4,00 g (28 %) farblosen Feststoff, R_{f}=0,42 (Dichlormethan/Methanol=95:5)
200 MHz ¹H-NMR (CDCl₃): 1.02, t, 3H; 1.56, t, 3H; 1.89, hex, 2H; 2.67, s, 3H; 3.00, t, 2H; 4.26, quart., 2H; 7.05, m, 2H; 7.50, dt, 1H; 8.17, dd, 1H; 10.00, s, 1H.

### Beispiel 5A

4-Ethoxy-3-(5-methyl-4-oxo-7-propyl-3,4-dihydro-imidazo[5,1-f][1,2,4]triazin-2-yl)-benzolsulfonsäurechlorid 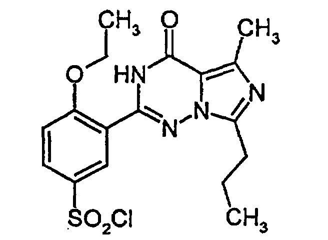 2,00 g (6,4 mmol) 2-(2-Ethoxy-phenyl)-5-methyl-7-propyl-3*H*-imidazo[5,1-f][1,2,4]-triazin-4-on werden langsam zu 3,83 ml Chlorsulfonsäure bei 0°C gegeben. Die Reaktionsmischung wird bei Raumtemperatur über Nacht gerührt, auf Eiswasser gegossen und mit Dichlormethan extrahiert. Man erhält 2,40 g (91 %) farblosen Schaum.

200 MHz ¹H-NMR (CDCl₃): 1.03, t, 3H; 1.61, t, 2H; 1.92, hex, 2H; 2.67, s, 3H; 3.10, t, 2H; 4.42, quart., 2H; 7.27, t, 1H; 8.20, dd, 1H; 8.67, d, 1H; 10.18, s, 1H.

### Beispiel 6A

(4-Piperidinylmethyl)-phosphonsäurediethylester

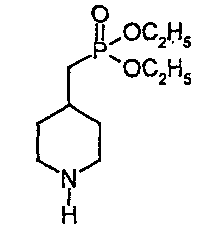

Man legt 2,11 g (528 mmol) 60%iges Natriumhydrid in 50ml absolutem Tetrahydrofuran vor und tropft 15,7 g (52,8 mmol) Methandiphosphonsäurediethylester hinzu. Man rührt noch 30 Minuten bei Raumtemperatur und tropft dann 10,1 g (52,8 mmol) 1-Benzyl-4-piperidon hinzu. Man rührt eine Stunde bei Raumtemperatur und eine Stunde unter Rückfluß, engt ein, versetzt mit Wasser, extrahiert dreimal mit Dichlormethan, trocknet über Natriumsulfat und engt ein. Der Rückstand wird in 50 ml Ethanol an 1,7 g 10%iger Palladium-Aktivkohle bei Raumtemperatur und 3 bar hydriert. Man saugt den Katalysator ab und engt das Filtrat ein.
Ausbeute: 12,5 g (100% d.Th.)
400 MHz, ¹H-NMR (CDCl₃): 1,13, m, 2H; 1,32, t, 6H; 1,69, dd, 2H; 1,74 - 1,95, m, 4H; 2,62, dt, 2H; 3,05, m, 2H; 4,1, m, 4H.

### Herstellungsbeispiele

### Beispiel 1

2-[2-Ethoxy-5-(4-methyl-piperazin-1-sulfonyl)-phenyl]-5-methyl-7-propyl-3H-imidazo[5,1-f][1,2,4]triazin-4-on 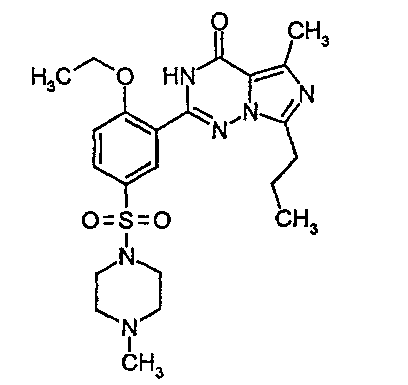 1,23 g (3 mmol) 4-Ethoxy-3-(5-methyl-4-oxo-7-propyl-3,4-dihydro-imidazo[5,1-f]-[1,2,4]triazin-2-yl)-benzolsulfonsäurechlorid werden in 40 ml Dichlormethan gelöst und auf 0°C gekühlt. Nach Zugabe einer Spatelspitze DMAP werden 0,90 g (9,00 mmol) N-Methylpiperazin zugegeben und die Reaktionsmischung über Nacht bei Raumtemperatur gerührt. Es wird mit Dichlormethan verdünnt, die organische Phase zweimal mit Wasser gewaschen, über Natriumsulfat getrocknet und das Lösungsmittel im Vakuum entfernt. Kristallisation aus Ether ergibt 1,25 g (88 %) farblosen Feststoff.
200 MHz ¹H-NMR (CDCl₃): 1.01, t, 3H; 1.59, t, 3H; 1.88, hex, 2H; 2.29, s, 3H; 2.51, m, 4H; 2.63, s, 3H; 3.00, t, 2H; 3.08, m, 4H; 4.33, quart., 2H, 7.17, d, ,1H; 7.88, dd, 1H; 8.44, d, 1H; 9.75, s, 1H.

### Beispiel 2

2-[2-Ethoxy-5-(4-ethyl-piperazin-1-sulfonyl)-phenyl]-5-methyl-7-propyl-3*H*-imidazo[5,1-f][1,2,4]triazin-4-on 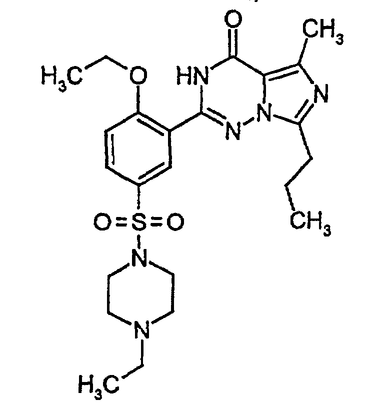 470 mg (1,14 mmol) 4-Ethoxy-3-(5-methyl-4-oxo-7-propyl-3,4-dihydro-imidazo-[5,1-f][1,2,4]triazin-2-yl)-benzolsulfonsäurechlorid werden in 20ml Dichlormethan gelöst und auf 0°C gekühlt. Es werden 390 mg (3,42 mmol) N-Ethylpiperazin zugegeben und die Reaktionsmischung über Nacht bei Raumtemperatur gerührt. Es wird mit Dichlormethan verdünnt, die organische Phase zweimal mit Wasser gewaschen, über Natriumsulfat getrocknet und das Lösungsmittel im Vakuum entfernt. Kristallisation aus Ether ergibt 370 mg (66 %) farblosen Feststoff.
400 MHz ¹H-NMR (CDCl₃). 1.01, t, 3H; 1.59, t, 3H; 1.88, hex, 2H; 2.42, quart., 2H; 2.56, m, 4H; 2.63, s, 3H; 3.00, t, 2H; 3.10, m, 4H; 4.33, quart., 2H, 7.17, d, ,1H; 7.88, dd, 1H; 8.44, d, 1H; 9.75, s, 1H.

### Beispiel 3

2-[2-Ethoxy-5-(4-hydroxy-piperidin-1-sulfonyl)-phenyl]-5-methyl-7-propyl-3*H*-imidazo[5,1-f][1,2,4]triazin-4-on 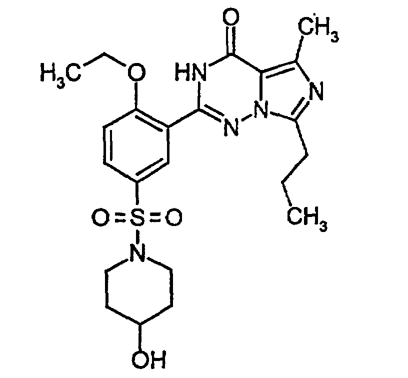 auf analoge Weise erhält man ausgehend von 531 mg (1,29 mmol) 4-Ethoxy-3-(5-methyl-4-oxo-7-propyl-3,4-dihydro-imidazo[5,1-f][1,2,4]triazin-2-yl)-benzolsulfonsäurechlorid und 393 mg (3,88 mmol) 4-Hydroxypiperidin 400 mg (64%) 2-[2-Ethoxy-5-(4-hydroxy-piperidin-1-sulfonyl)-phenyl]-5-methyl-7-propyl-3*H*-imidazo[5,1-f][1,2,4]triazin-4-on
200 MHz ¹H-NMR (DMSO-d6): 0.941, t, 3H; 1.32, t, 3H; 1.45, m, 2H; 1.71, m, 4H; 2.48, s, 3H; 2.82, m, 4H; 3.11,m, 2H; 3.55, m, 1H; 4.20, quart., 2H; 4.72, d, 1H, 7.39, d,1H; 7.87, m, 2H; 11.70, s, 1H.

### Beispiel 4

2-{2-Ethoxy-5-[4-(2-hydroxy-ethyl)-piperazin-1-sulfonyl]-phenyl}-5-methyl-7-propyl-3*H*-imidazo[5,1-f][1,2,4]triazin-4-on 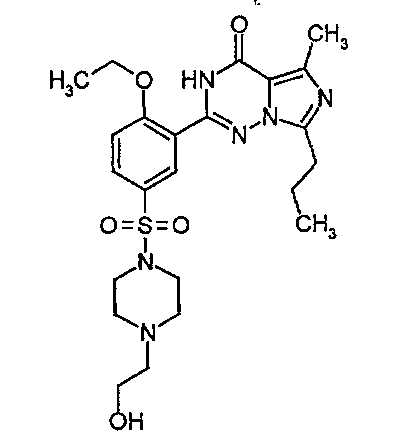 auf analoge Weise erhält man ausgehend von 411 mg (1 mmol) 4-Ethoxy-3-(5-methyl-4-oxo-7-propyl-3,4-dihydro-imidazo[5,1-f][1,2,4]triazin-2-yl)-benzolsulfonsäurechlorid und 391 mg (3 mmol) 4-Hydroxyethylpiperazin 380 mg (75 %) 2-{2-Ethoxy-5-[4-(2-hydroxy-ethyl)-piperazin-1-sulfonyl]-phenyl}-5-methyl-7-propyl-3*H*-imidazo[5,1-f][1,2,4]triazin-4-on
R_{f}=0.198 (Dichlormethan/Methanol=95:5)
200 MHz ¹H-NMR (CDCl₃):1.02, t, 3H; 1.61, t, 3H; 1.87, hex., 3H; 2.60, m, 7H; 3.00, t, 2H; 3.10, m, 4H; 3.60, t, 2H; 4.36, quart., 2H; 7.18, d, 1H, 7.89, dd, 1H, 8.47, d, 1H, 9.71, s, 1H.

### Beispiel 5

N,N-Diethyl-4-ethoxy-3-(5-methyl-4-oxo-7-propyl-3,4-dihydro-imidazo[5,1-f][1,2,4]triazin-2-yl)benzolsulfonamid 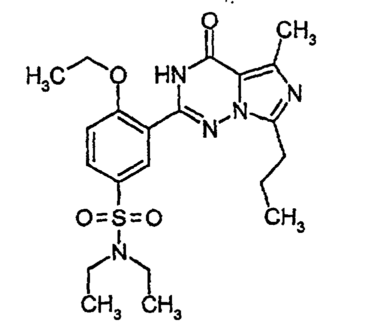 auf analoge Weise erhält man ausgehend von 400 mg (0,97 mmol) 4-Ethoxy-3-(5-methyl-4-oxo-7-propyl-3,4-dihydro-imidazo[5,1-f][1,2,4]triazin-2-yl)-benzolsulfonsäurechlorid und 210 mg (2,92 mmol) Diethylamin 398 mg (89 %) N,N-Diethyl-4-ethoxy-3-(5-methyl-4-oxo-7-propyl-3,4-dihydro-imidazo[5,1-f][1,2,4]triazin-2-yl)benzolsulfonamid
R_{f}=0.49 (Dichlormethan/Methanol=20:1)
200 MHz ¹H-NMR (CDCl₃):1.02, t, 3H; 1.20, t, 6H; 1.49, t, 1.61, t, 3H; 1.88, sex., 2H; 2.30, s, breit, 1H; 2.62, s, 3H; 2.99, t, 2H; 3.32, m, 4H; 3.78, t, 2H; 3.80, m, 2H; 4.37, quart., 2H; 7.15, d, 1H; 7.98, dd, 1H; 8.56, d, 1H; 9.70, s, 1H.

### Beispiel 6

2-[2-Ethoxy-5-(4-methylpiperazin-1-sulfonyl)-phenyl]-5-ethyl-7-propyl-3*H*-imidazo[5,1-f]-[1,2,4]triazin-4-on

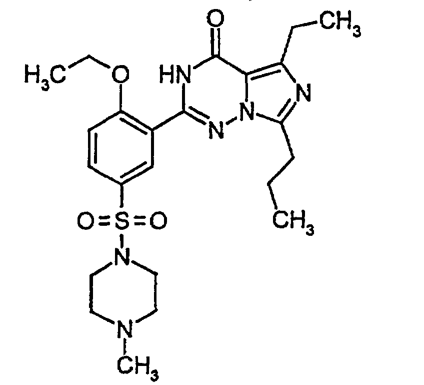

Auf analoge Weise erhält man ausgehend von 640 mg (1,50 mmol) 4-Ethoxy-3-(5-ethyl-4-oxo-7-propyl-3,4-dihydro-imidazo[5,1-f][1,2,4]triazin-2-yl)-benzolsulfonsäurechlorid und 450 mg (4,5 mmol) 4-Hydroxyethylpiperazin 495 mg (66 %) 2-[2-Ethoxy-5-(4-methylpiperazin-1-sulfonyl)-phenyl]-5-ethyl-7-propyl-3*H*-imidazo[5,1-f][1,2,4]triazin-4-on.
R_{f}=0.30(Dichlormethan/Methanol=19:1)
200 MHz ¹H-NMR (CDCl₃):1.01, t, 3H; 1.35, t, 3H; 1.61, t, 3H; 1.89, sex., 2H; 2.31, s, 3H; 2.53, m, 4H; 3.05, m, 8H; 4.35, quart., 2H; 7.17, d, 1H; 7.89, dd, 1H; 8.48, d, 1H; 9.65, s, 1H.

Die im folgenden aufgeführten Sulfonamide wurden mittels automatisierter Parallelsynthese aus 4-Ethoxy-3-(5-methyl-4-oxo-7-propyl-3,4-dihydro-imidazo[5,1-f][1,2,4]triazin-2-yl)-benzolsulfonsäurechlorid und dem entsprechenden Amin nach einer der drei folgenden Standardvorschriften hergestellt.

Die Reinheit der Endprodukte wurde mittels HPLC bestimmt, ihre Charakterisierungen durch LC-MS Messung vorgenommen. Der Gehalt der gewünschten Verbindung nach HPLC-MS ist in den Tabellen in der Spalte "HPLC" in Prozent angegeben. Standardvorschrift A wurde angewendet bei Aminen mit aciden Funktionalitäten, Standardvorschrift B bei Aminen mit neutralen Funktionalitäten, Standardvorschrift C bei Aminen mit zusätzlichen basischen Funktionalitäten.

In den folgenden Strukturformeln wurde gelegentlich auf die Abbildung der Wasserstoffatome verzichtet. Stickstoffatome mit einer freien Valenz sind daher als-NH-Rest zu verstehen.

Standardvorschrift A: Umsetzung von Aminen mit aciden Funktionalitäten Zunächst werden 0,05 mmol Amin, 0,042 mmol Sulfonsäurechlorid und 0,10 mmol Na₂CO₃ vorgelegt und 0,5 ml eines Gemisches aus THF/H₂O von Hand zupipettiert. Nach 24 h bei RT wird mit 0,5 ml 1 M H₂SO₄-Lösung versetzt und über eine zweiphasige Kartusche filtrier (500 mg Extrelut (Oberphase) und 500 mg SiO₂, Laufmittel Essigester). Nach dem Einengen des Filtrates im Vakuum erhält man das Produkt.

Standardvorschrift B: Umsetzung von Aminen mit neutralen Funktionalitäten Zunächst werden 0,125 mmol Amin vorgelegt und vom Synthesizer 0,03 mmol Sulfonsäurechlorid als Lösung in 1,2-Dichlorethan zupipettiert. Nach 24 h wird das Gemisch mit 0,5 ml 1 M H₂SO₄ versetzt und über eine zweiphasige Kartusche (500 mg Extrelut (Oberphase) und 500 mg SiO₂, Laufinittel: Essigester) filtriert. Das Filtrat wird im Vakuum eingeengt.

Standardvorschrift C: Umsetzung von Aminen mit basischen Funktionalitäten Zunächst werden 0,05 mmol Amin vorgelegt und vom Synthesizer 0,038 mmol Sulfonsäurechlorid als Lösung in 1,2-Dichlorethan und 0,05 mmol Triethylamin als Lösung in 1,2-Dichlorethan zupipettiert. Nach 24 h wird zunächst mit 3 ml gesättigter NaHCO₃-Lösung versetzt und das Reaktionsgemisch über eine zweiphasige Kartusche filtriert. Nach dem Einengen des Filtrates im Vakuum erhält man das Produkt.

Alle Reaktionen werden dünnschichtchromatographisch verfolgt. Für den Fall, daß nach 24 h bei RT keine vollständige Umsetzung erfolgt ist, wird für weitere 12 h auf 60°C erhitzt und im Anschluß der Versuch beendet.

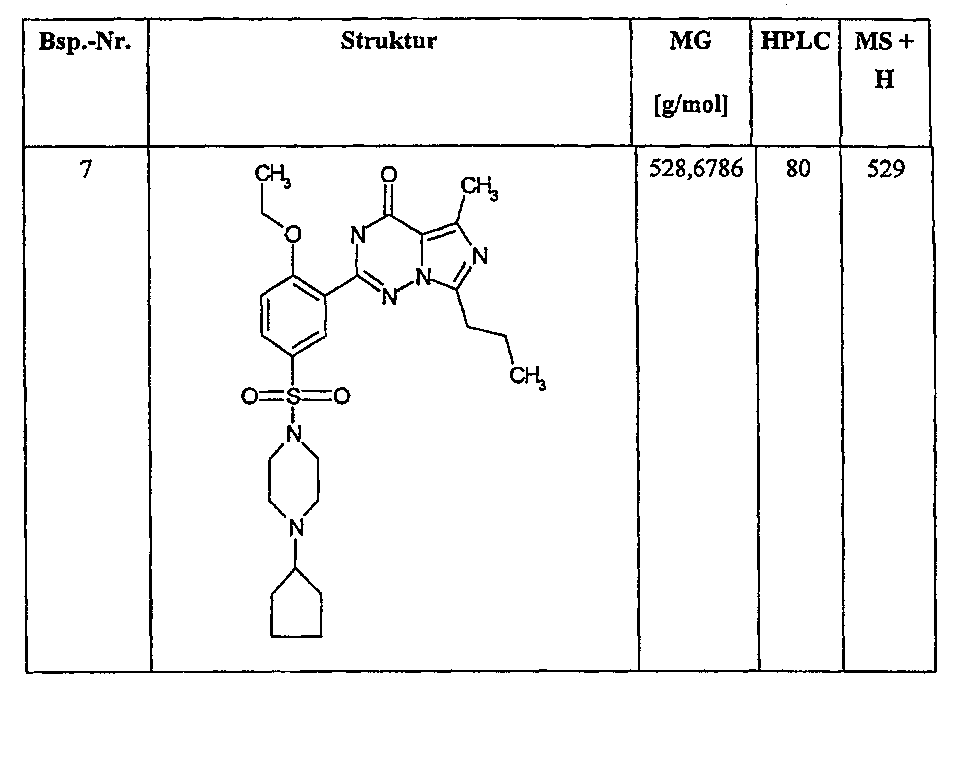

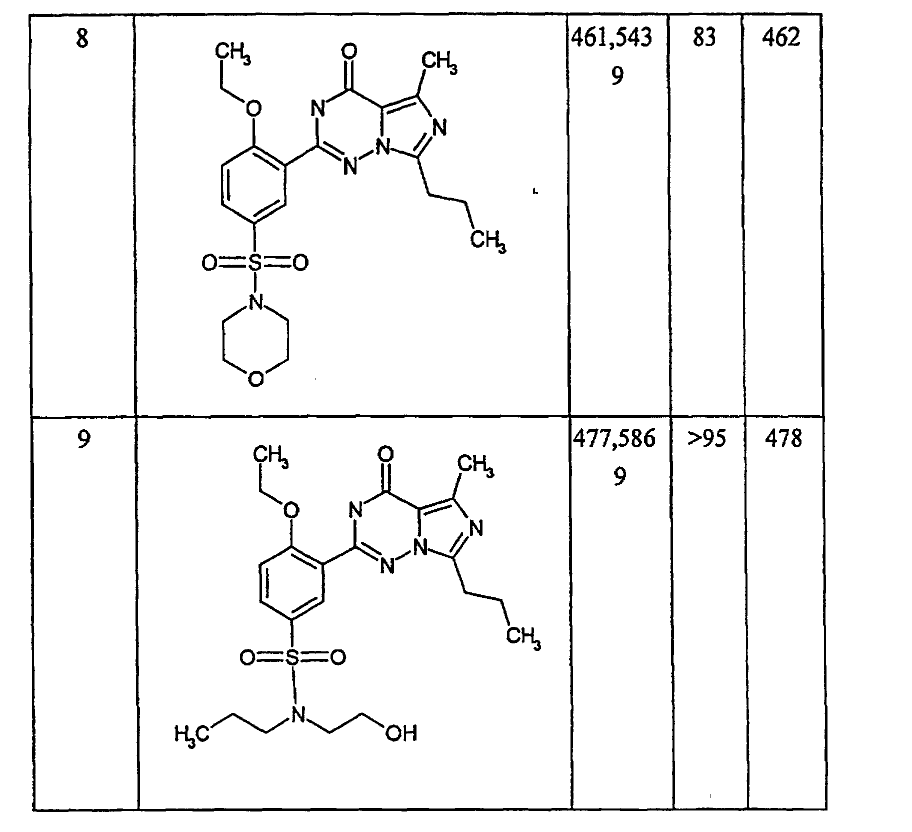

### Beispiel 10

2-[2-Ethoxy-5-(4-ethyl-piperazin-1-sulfonyl)-phenyl]-5-methyl-7-propyl-3H-imidazo[5,1-f][1,2,4]triazin-4-on Dihydrochlorid 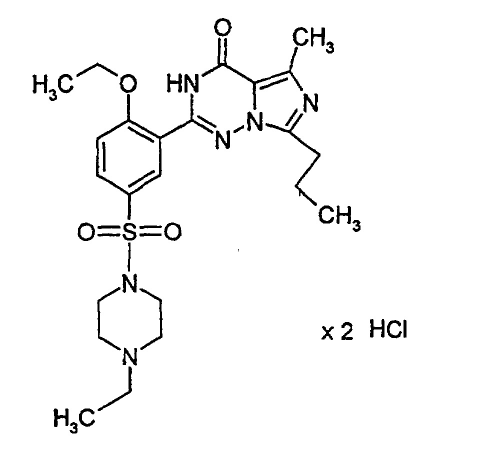 0,35 g (0,712 mmol) 2-[2-Ethoxy-5-(4-ethyl-piperazin-1-sulfonyl)-phenyl]-5-methyl-7-propyl-3H-imidazo[5,1-f][1,2,4]triazin-4-on werden in 8 ml Ether suspendiert und soviel Dichlormethan zugegeben, bis eine homogene Lösung entsteht. Man gibt 2,4 ml einer 1M Lösung von HCl in Ether zu, rührt 20 Minuten bei Raumtemperatur und saugt ab. Man erhält 372 mg (99 %) 2-[2-Ethoxy-5-(4-ethyl-piperazin-1-sulfonyl)-phenyl]-5-methyl-7-propyl-3H-imidazo[5,1-f][1,2,4]triazin-4-on Dihydrochlorid.
200 Mhz ¹H-NMR (DMSO-d₆): 0,96, t, 3H; 1,22, t, 3H; 1,36, t, 3H; 1,82, sex., 2H; 2,61, s, 3H; 2,88, m, 2H; 3,08, m, 6H; 3,50, m, 2H; 3,70, m, 2H; 4,25, quart., 2H; 7,48, d, 1H; 7,95, m, 2H; 11,42, s, 1H; 12,45, s, 1H.

### Beispiel 11

2-[2-Ethoxy-5-(4-ethyl-piperazin-1-sulfonyl)-phenyl]-5-methyl-7-propyl-3H-imidazol [5,1-f][1,2,4]triazin-4-on Hydrochlorid-Trihydrat

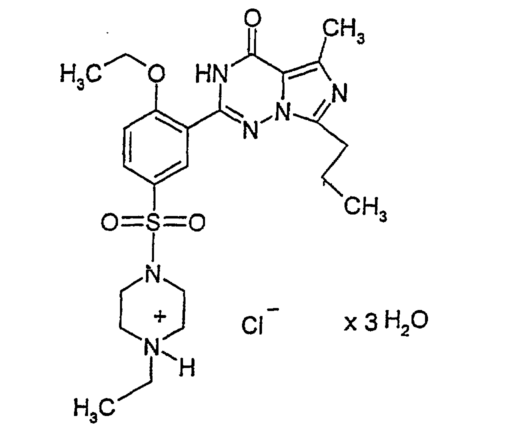

Kristallisiert man die freie Base aus Beispiel 2 aus einem Gemisch eines organischen Lösungsmittels und verdünnter wäßriger Salzsäure um, so erhält man ein Hydrochlorid Trihydrat.
Fp.: 218°C
Wassergehalt: 9,4 % (K. Fischer)
Chloridgehalt: 6,1 %

## Patentansprüche

1. Verbindung ausgewählt aus der folgenden Tabelle A: 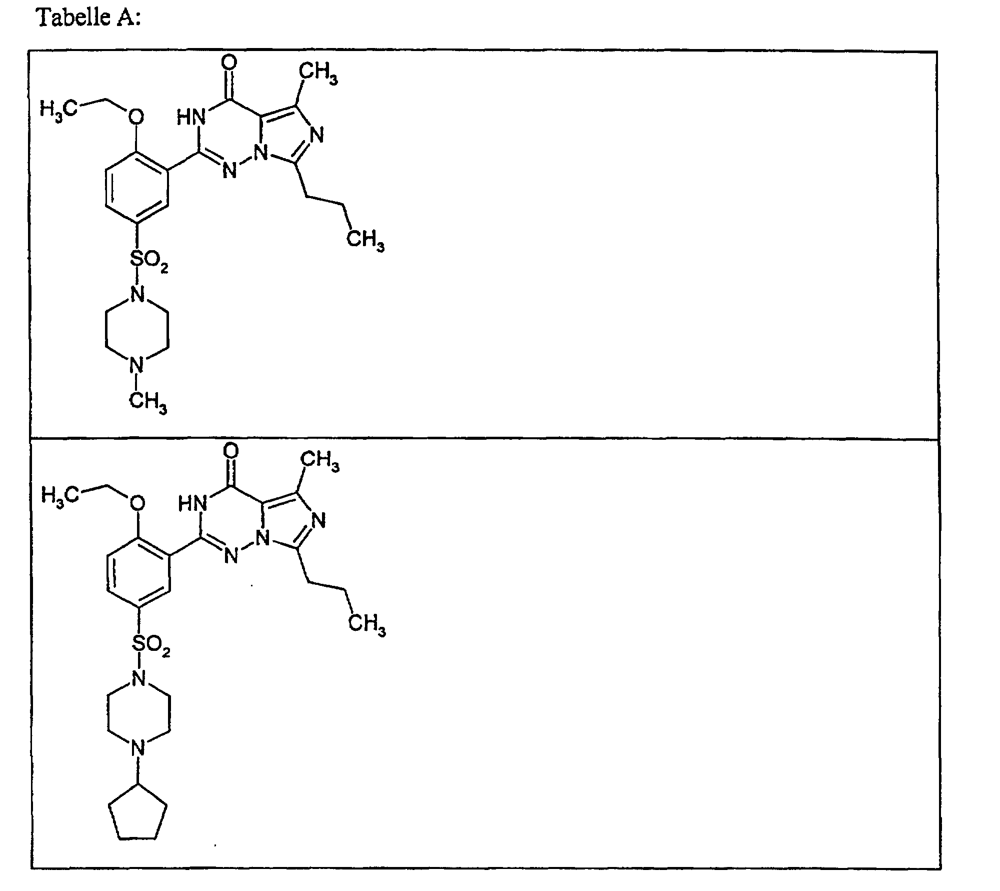 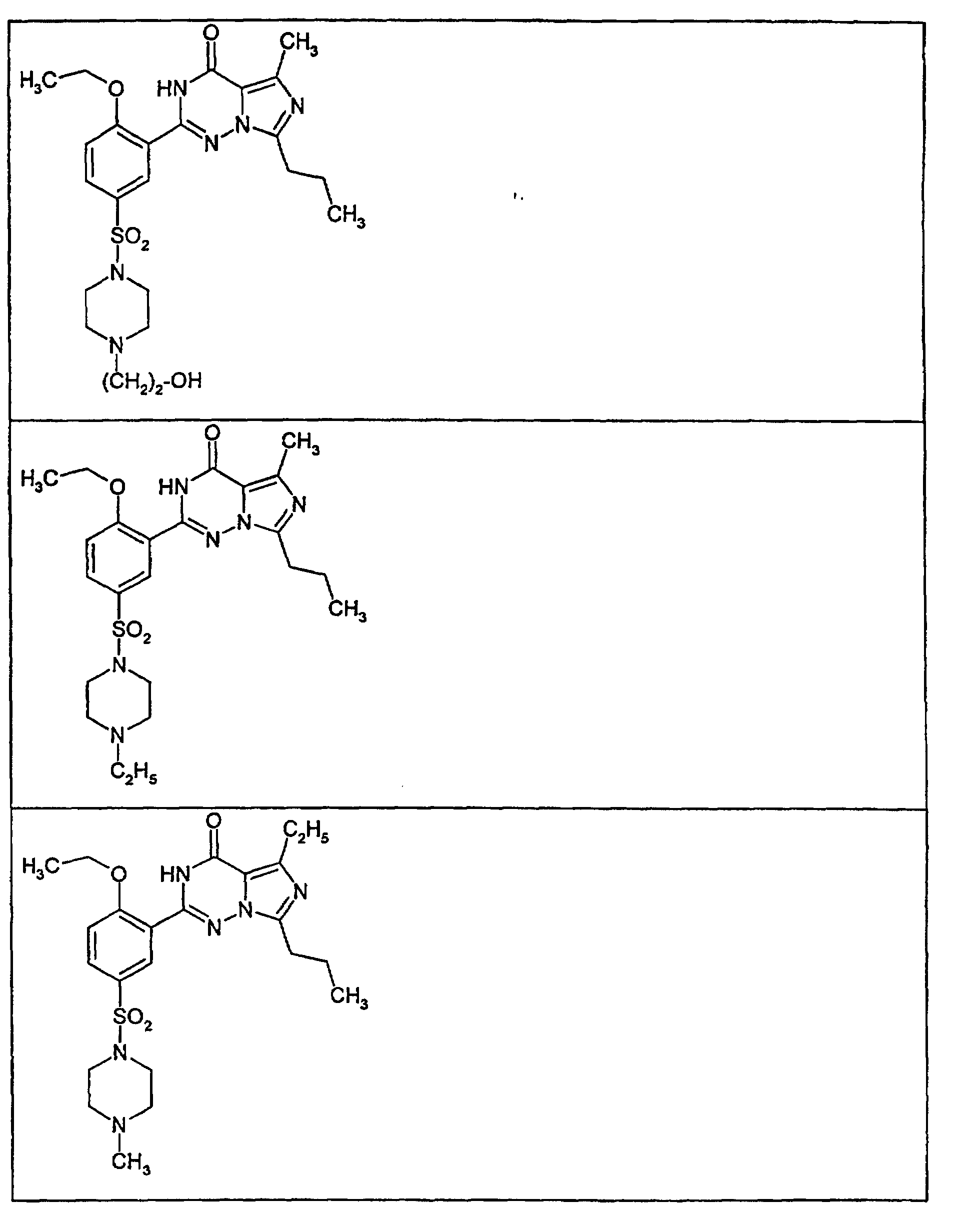 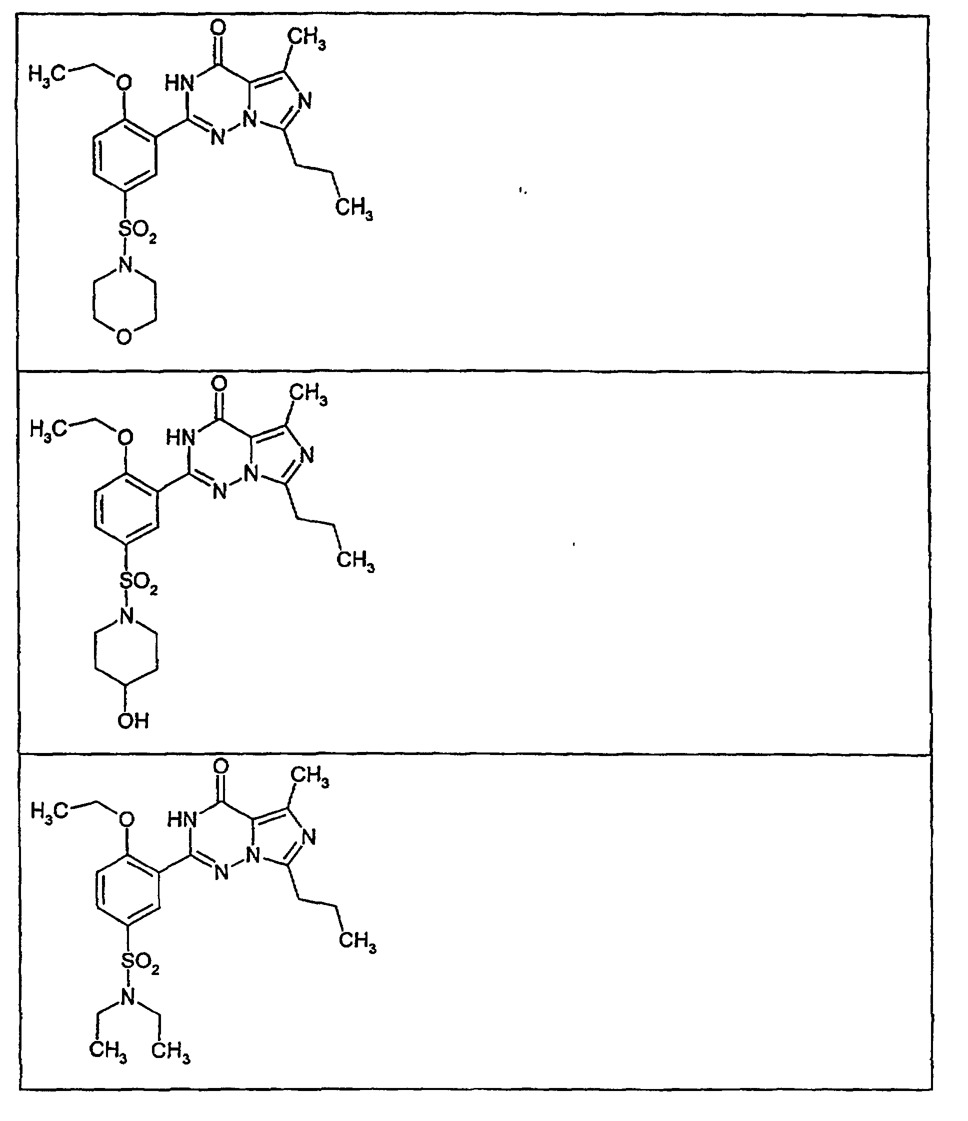 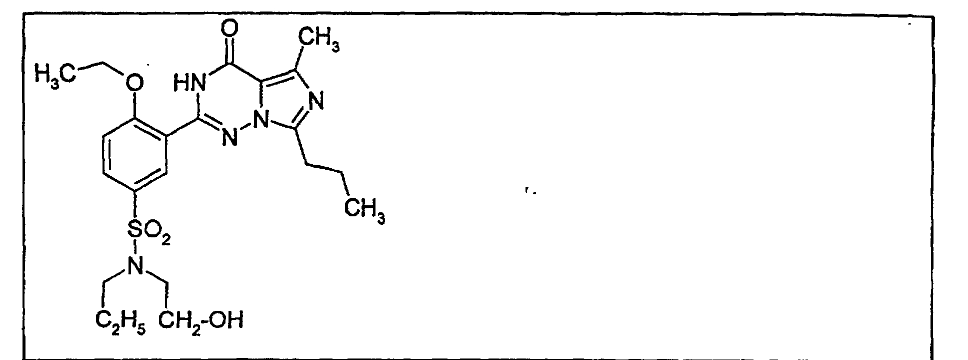 und deren physiologisch unbedenkliche Salze und Hydrate.

2. Verbindung gemäß der folgenden Formel:

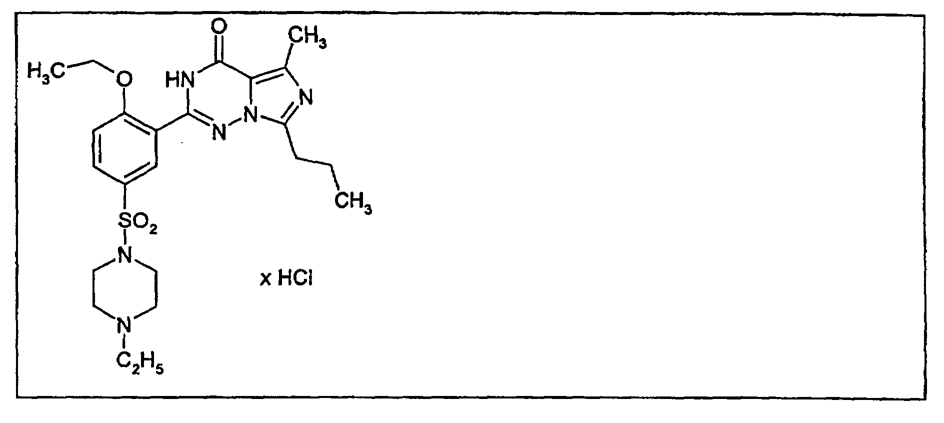

3. Verbindung gemäß der folgenden Formel:

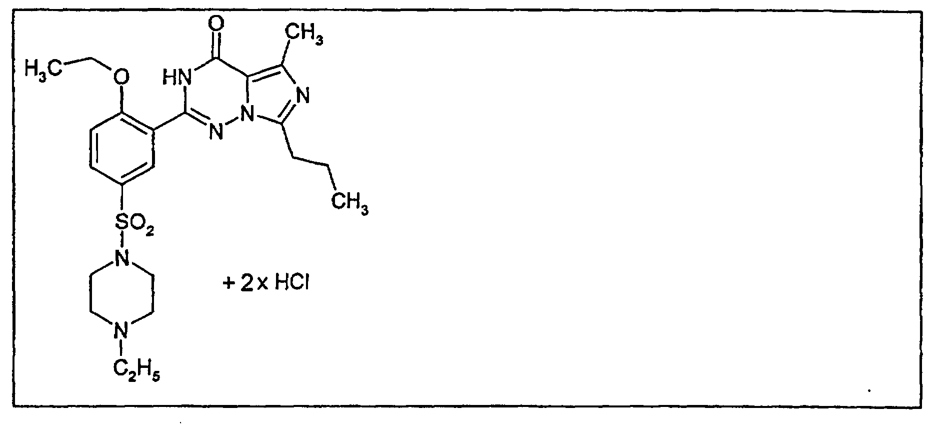

4. Verbindung gemäß der folgenden Formel:

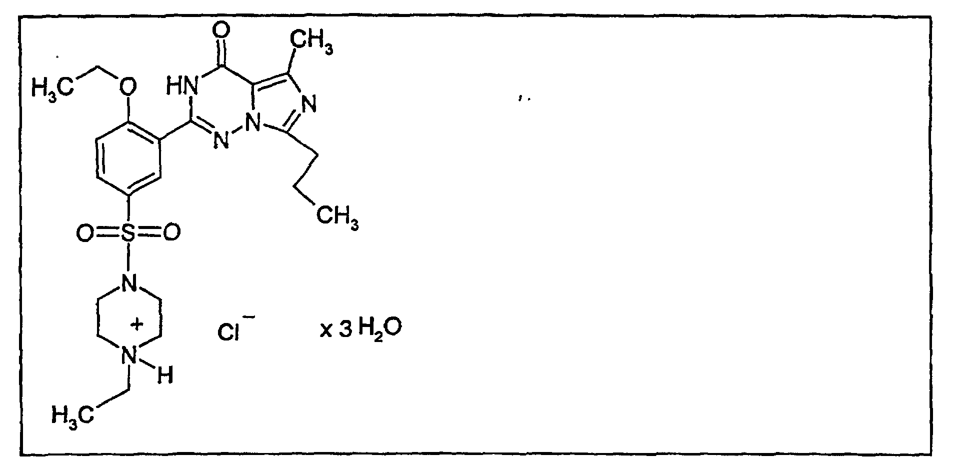

5. Verbindung gemäß einem der Ansprüche 1 bis 4 zur Behandlung von Erkrankungen.

6. Verfahren zur Herstellung von Verbindungen gemäß einem der Ansprüche 1 bis 4, **dadurch gekennzeichnet, daß** man
zunächst Verbindungen der allgemeinen Formel (II) 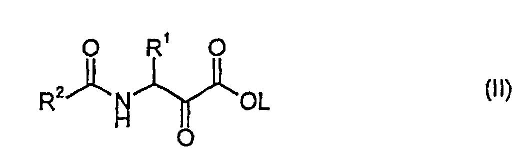 in welcher
R¹ für Methyl oder Ethyl und R² für Propyl steht
und
L für geradkettiges oder verzweigtes Alkyl mit bis zu 4 Kohlenstoffatomen steht,
mit Verbindungen der allgemeinen Formel (III) 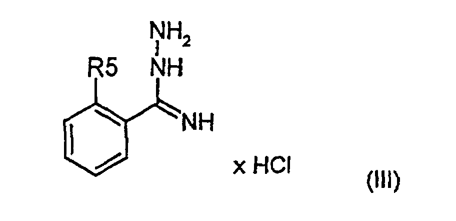 in der R⁵ für einen Ethoxyrest steht.
in einer Zweistufenreaktion in den Systemen Ethanol und Phosphoroxytrichlorid Dichlorethan in die Verbindungen der allgemeinen Formel (IV) 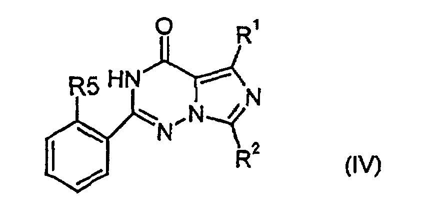 in welcher
R¹, R² und R⁵ die oben angegebene Bedeutung haben,
überführt, in einem weiteren Schritt mit Chlorsulfonsäure zu den Verbindungen der allgemeinen Formel (V) 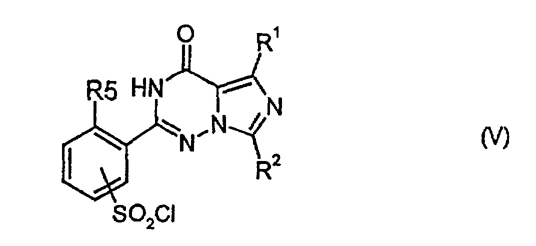 in welcher
R¹, R² und R⁵, die oben angegebene Bedeutung haben,
umsetzt und abschließend mit den entsprechenden Aminen in inerten Lösemitteln umsetzt.

7. Arzneimittel enthaltend mindestens eine Verbindung gemäß einem der Ansprüche 1 bis 4 sowie pharmakologisch unbedenkliche Formulierungsmittel.

8. Arzneimittel gemäß Anspruch 7 zur Behandlung von cardiovaskulären, cerebrovaskulären Erkrankungen und/oder Erkrankungen des Urogenitaltraktes.

9. Arzneimittel gemäß Anspruch 8 zur Behandlung von erektiler Dysfunktion.

10. Verwendung von Verbindungen gemäß einem der Ansprüche 1 bis 4 zur Herstellung von Arzneimitteln.

11. Verwendung gemäß Anspruch 10, wobei das Arzneimittelgegen erektile Dysfunktion wirkt.

## Claims

1. Compound selected from Table A below: 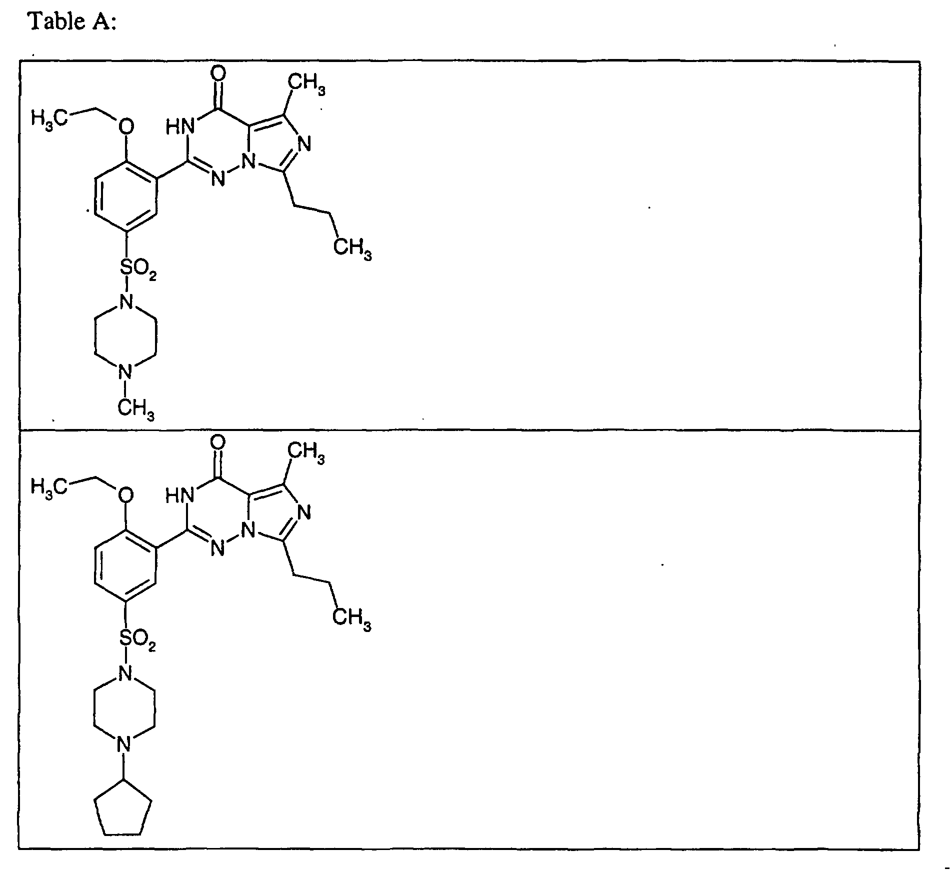 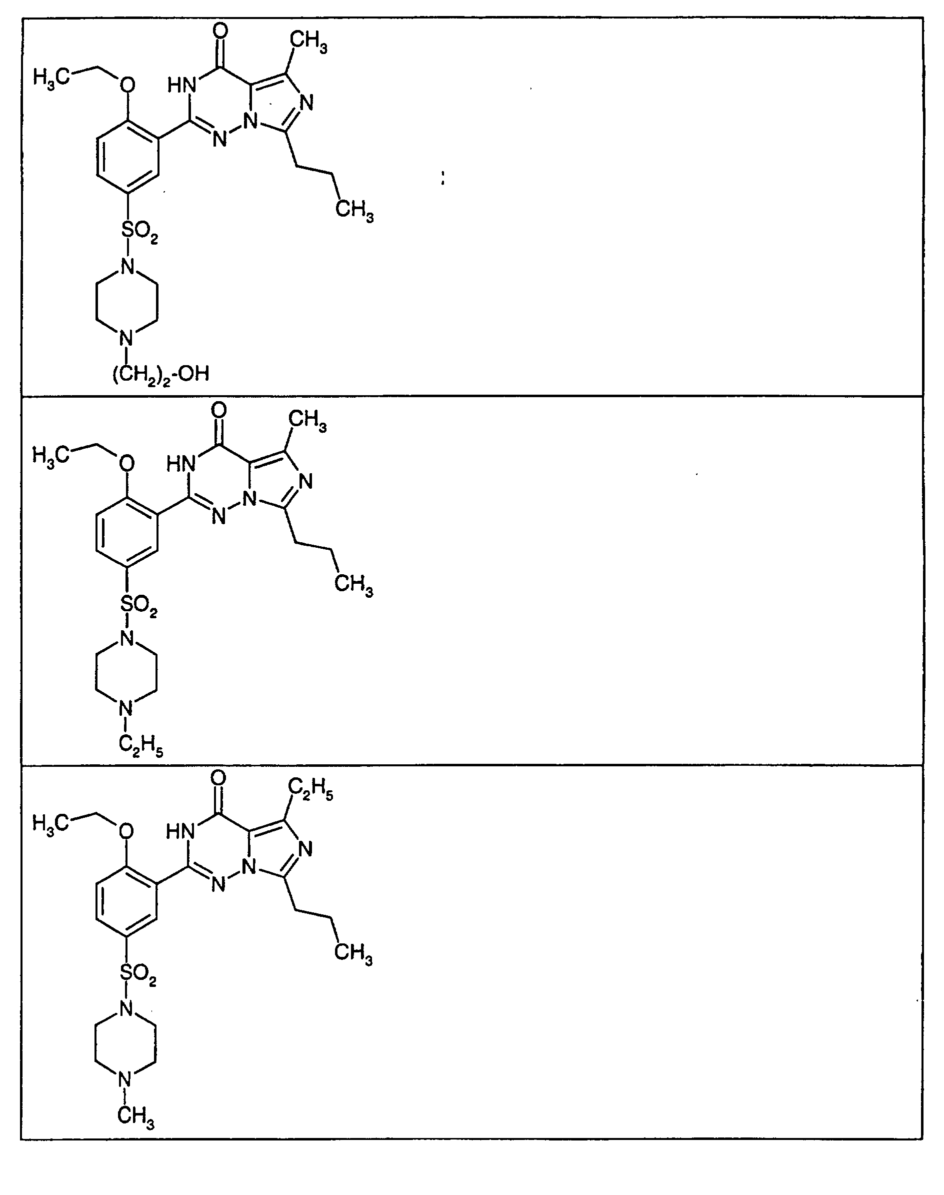 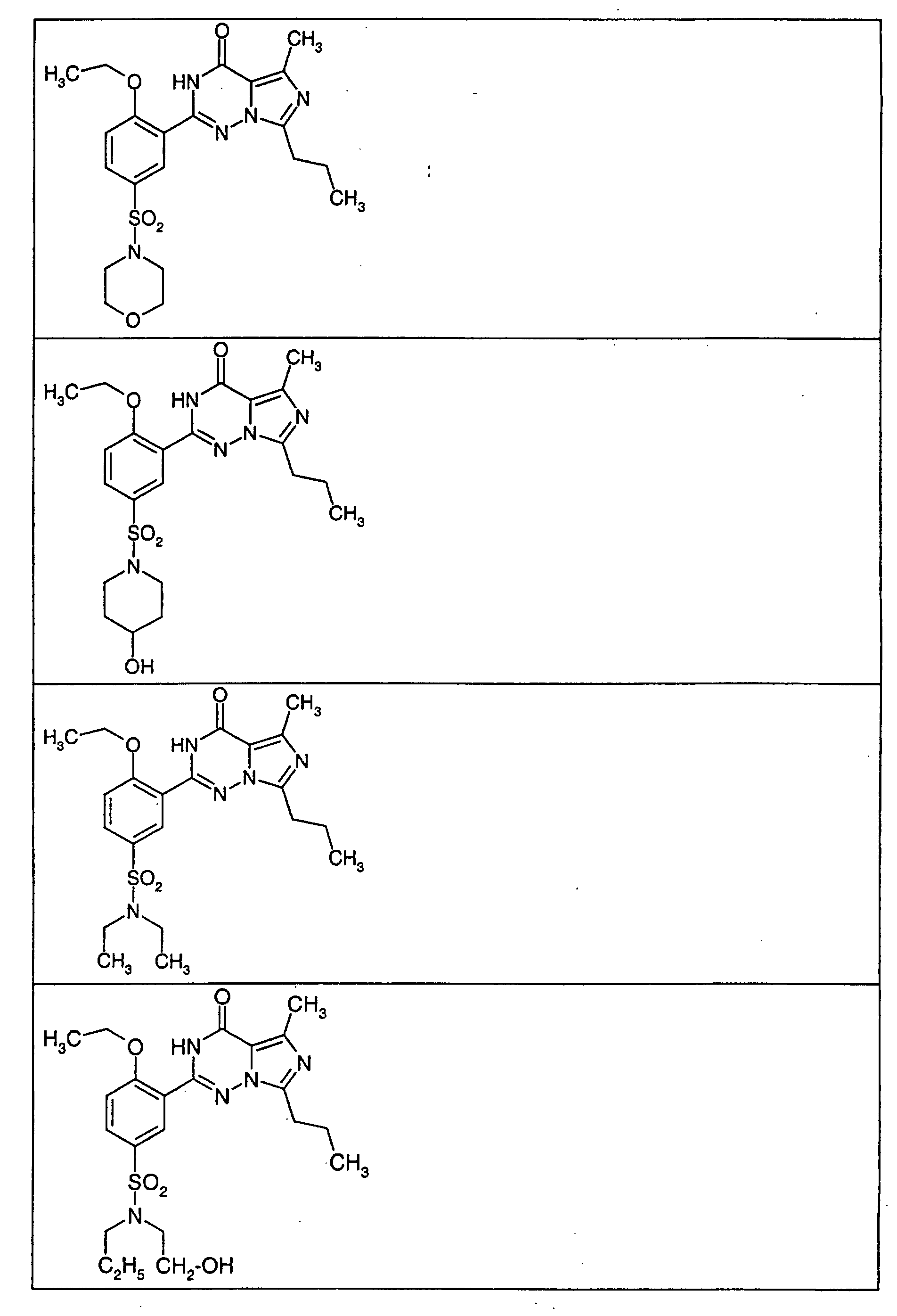 and their physiologically acceptable salts and hydrates.

2. Compound of the following formula:

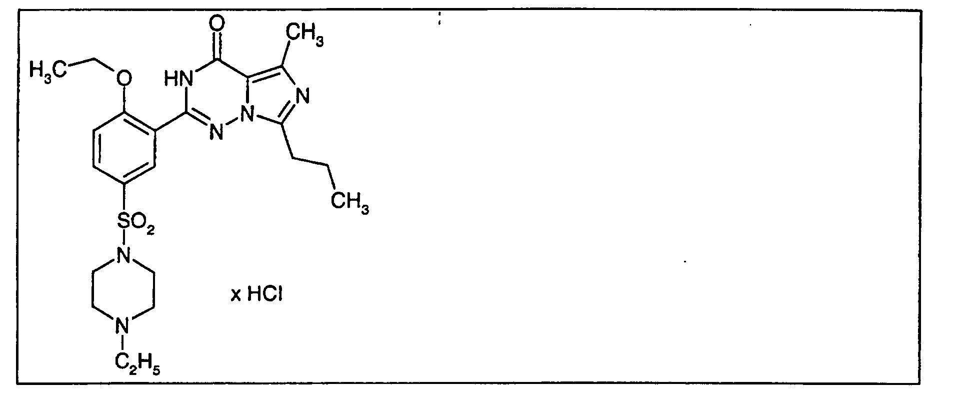

3. Compound of the following formula:

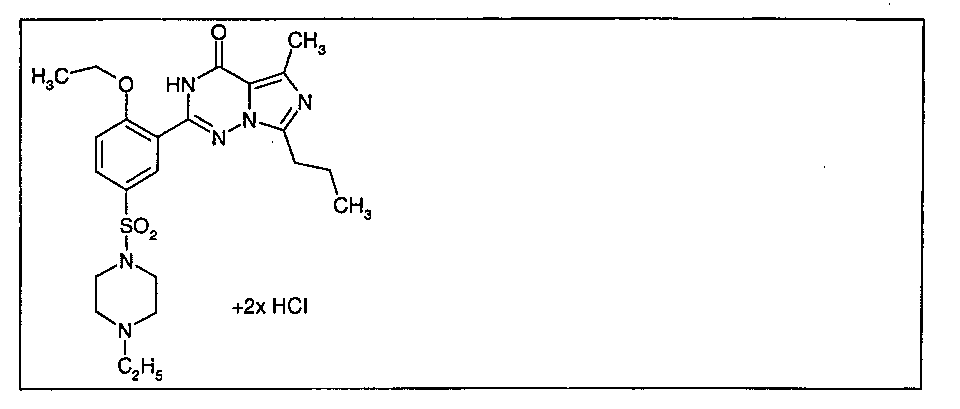

4. Compound of the following formula:

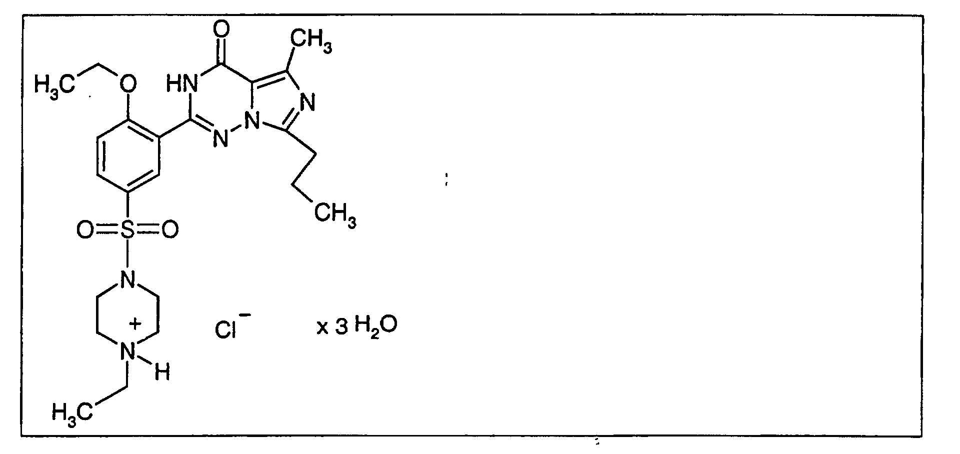

5. Compound according to one of Claims 1 to 4 for the treatment of disorders.

6. Process for preparing compounds according to one of Claims 1 to 4, **characterized in that**
initially compounds of the general formula (II) 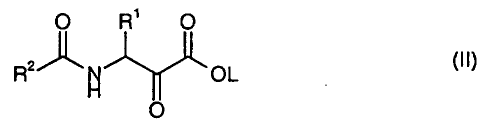 in which
R¹ represents methyl or ethyl and R² represents propyl
and
L represents straight-chain or branched alkyl having up to 4 carbon atoms,
are convened with compounds of the general formula (III) 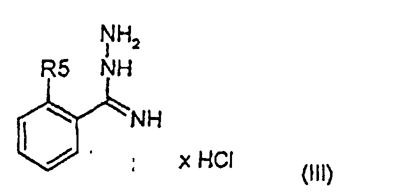 in which R⁵ represents an ethoxy radical,
in a two-step reaction in the systems ethanol and phosphorus oxytrichloride/ dichloroethane into the compounds of the general formula (IV) 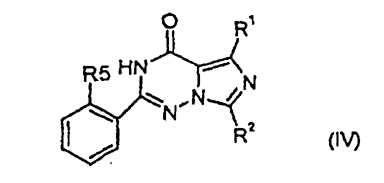 in which
R¹, R² and R⁵ are each as defined above,
which are reacted in a further step with chlorosulphonic acid to give the compounds of the general formula (V) 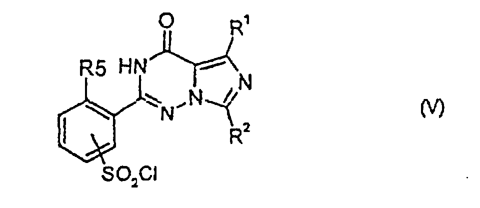 in which
R¹, R² and R⁵ are each as defined above,
which are finally reacted with the corresponding amines in inert solvents.

7. Pharmaceuticals which comprise at least one compound according to one of Claims 1 to 4 and pharmacologically acceptable formulating agents.

8. Pharmaceuticals according to Claim 7 for the treatment of cardiovascular, cerebrovascular disorders and/or disorders of the urogenital tract.

9. Pharmaceuticals according to Claim 8 for the treatment of erectile dysfunction.

10. Use of compounds according to one of Claims 1 to 4 for preparing pharmaceuticals.

11. Use according to Claim 10, the pharmaceutical acting against erectile dysfunction.

## Revendications

1. Composé choisi dans le Tableau A suivant : 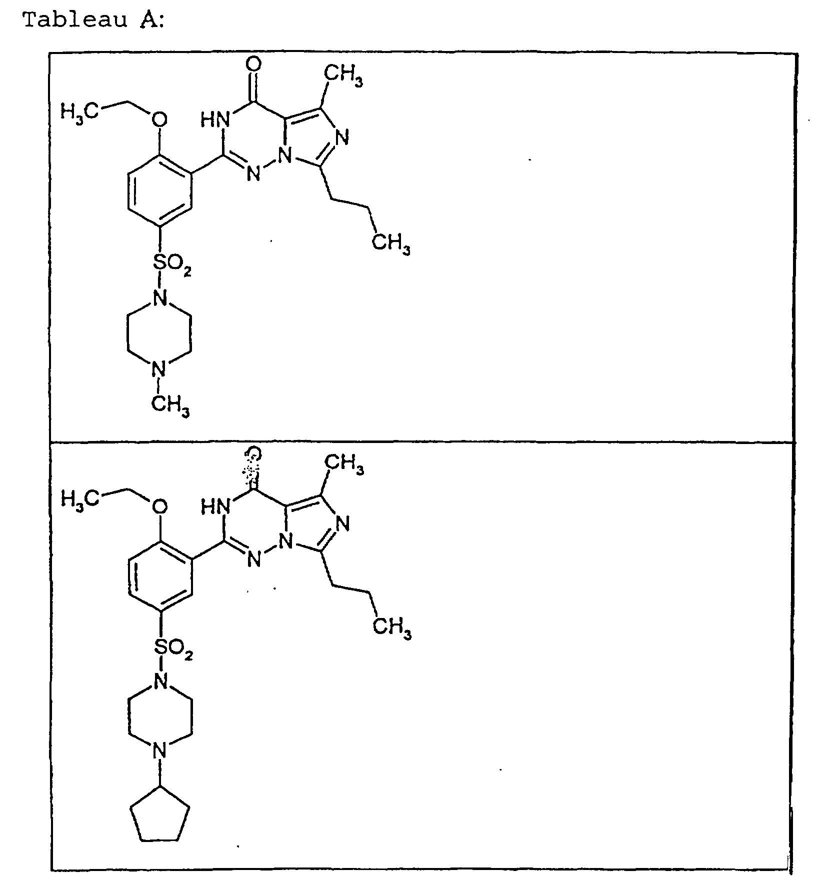 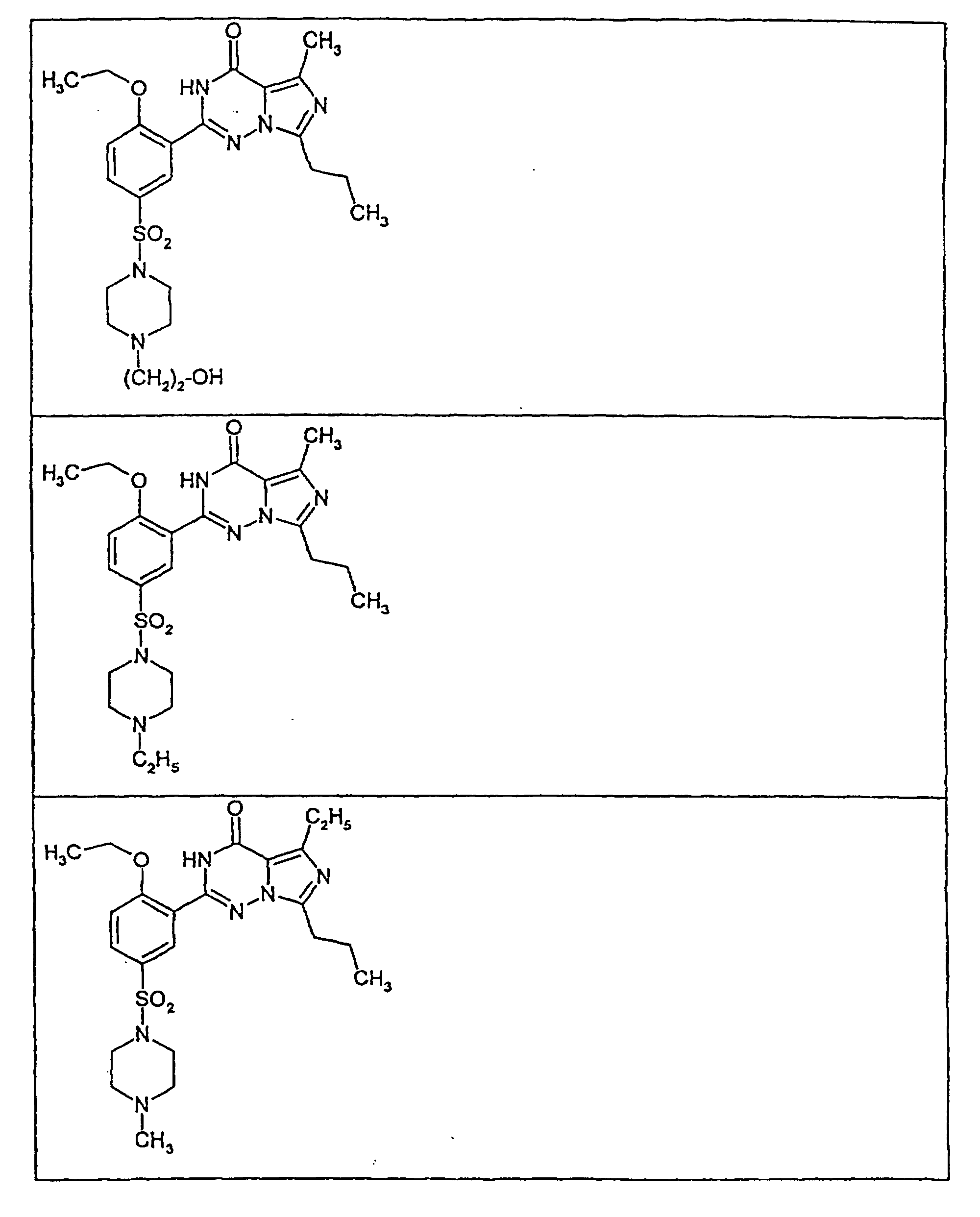 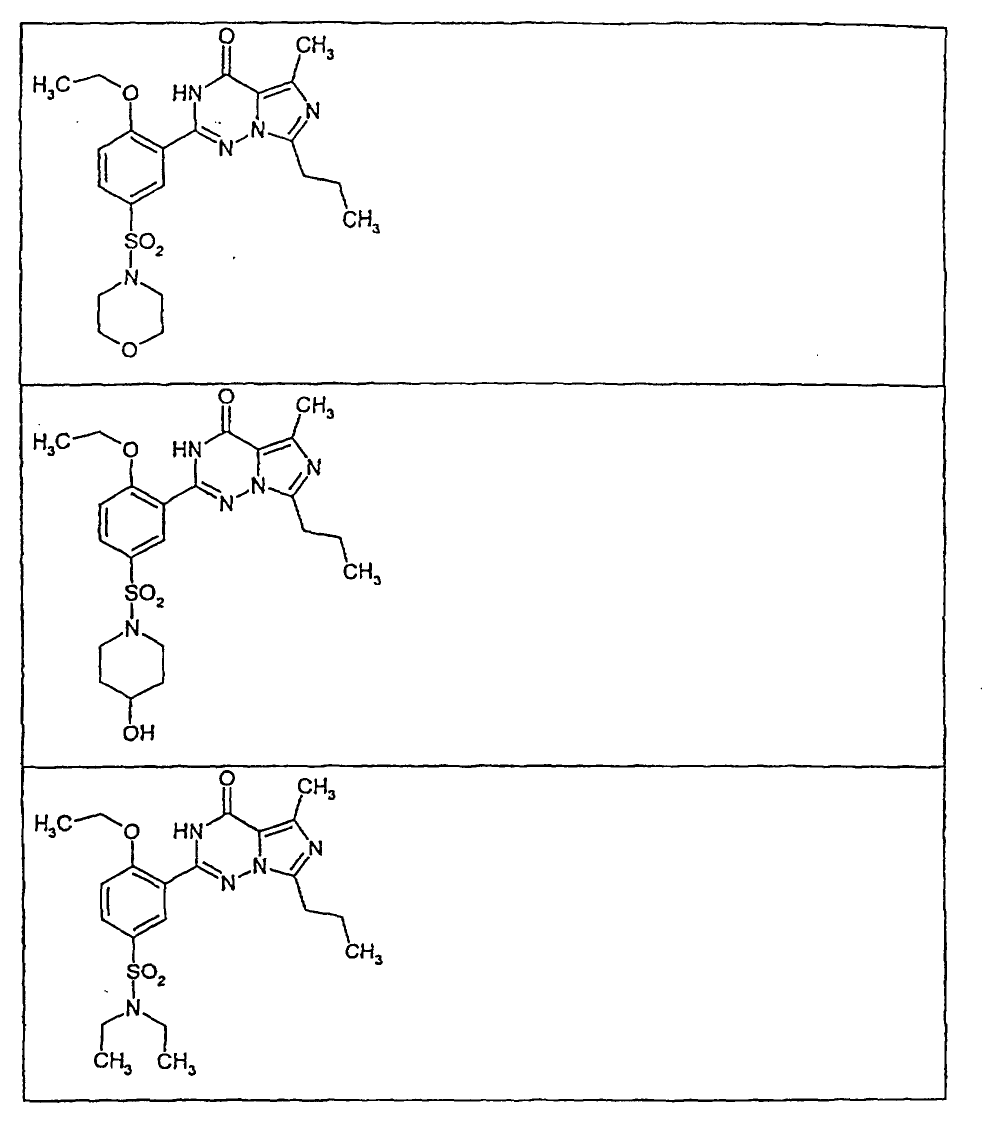 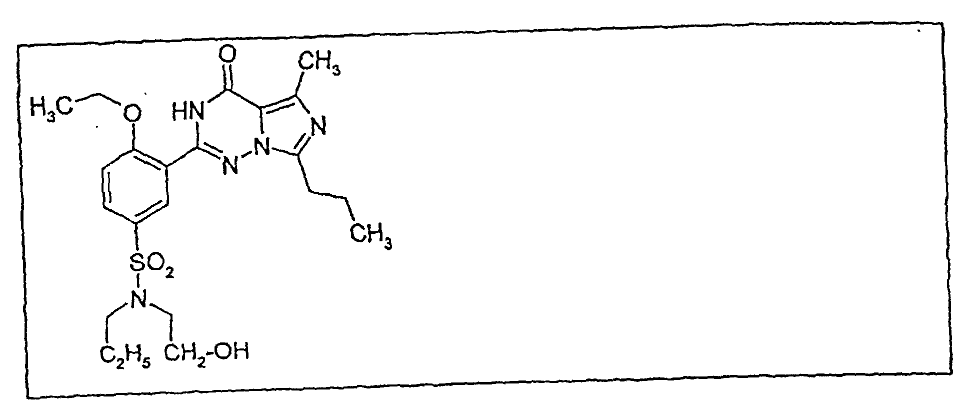 et ses sels et hydrates acceptables du point de vue physiologique.

2. Composé répondant à la formule suivante :

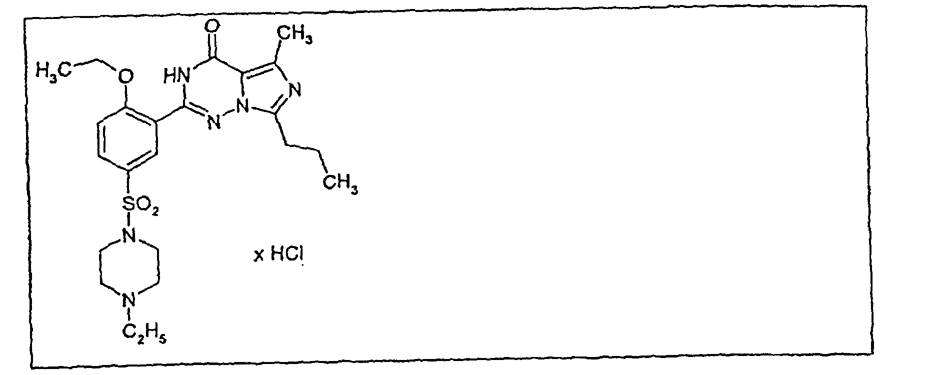

3. Composé répondant à la formule suivante :

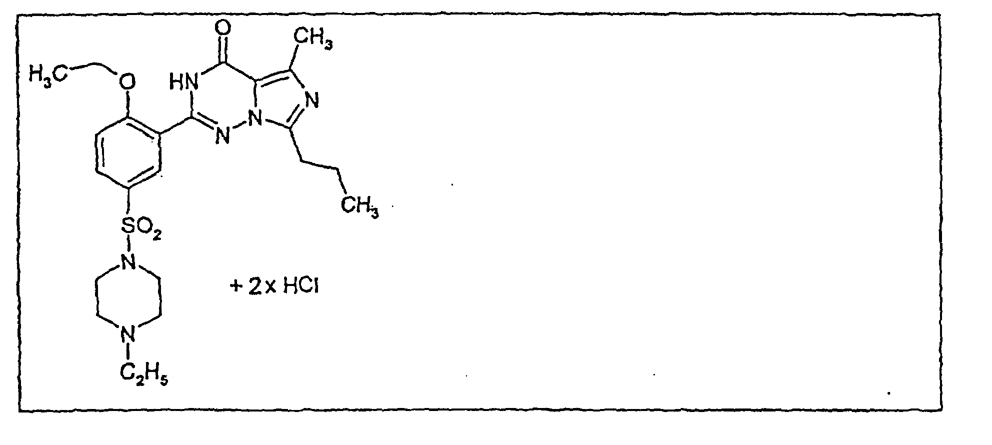

4. Composé répondant à la formule suivante :

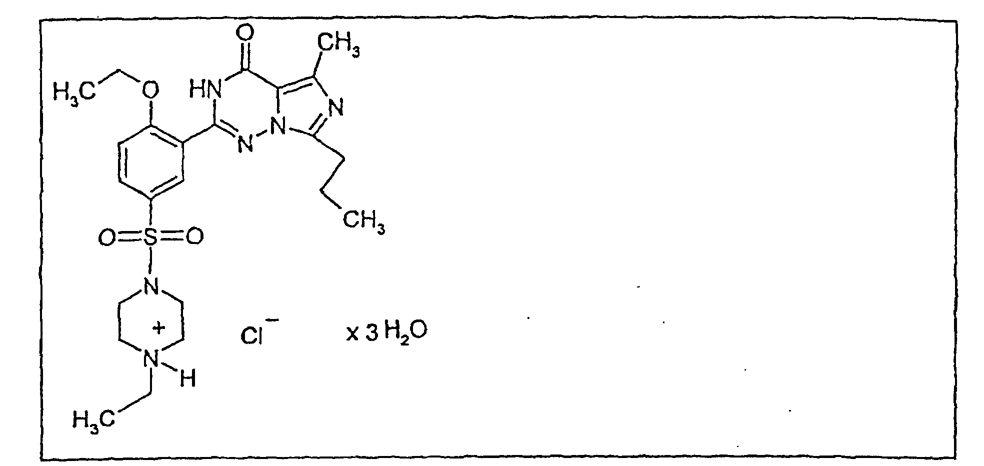

5. Composé suivant l'une des revendications 1 à 4, destiné au traitement de maladies.

6. Procédé de production de composés suivant l'une des revendications 1 à 4, **caractérisé en ce qu'**on transforme tout d'abord des composés de formule générale (II) 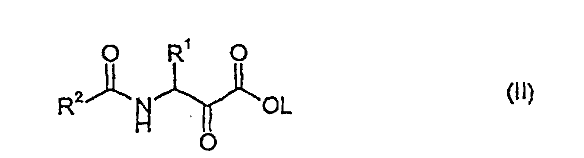 dans laquelle
R¹ est le groupe méthyle ou éthyle et R² est le groupe propyle
et
L représente un groupe alkyle linéaire ou ramifié ayant jusqu'à 4 atomes de carbone,
avec des composés de formule générale (III) 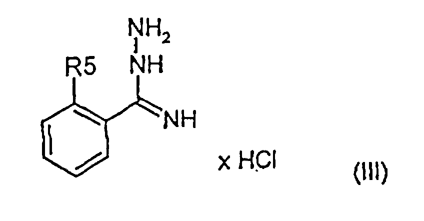 dans laquelle R⁵ représente un reste éthoxy,
dans une réaction en deux étapes dans les systèmes éthanol et oxytrichlorure de phosphore/dichloréthane, en les composés de formule générale (IV) 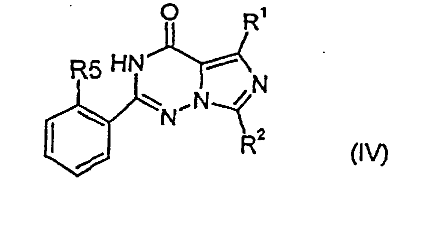 dans laquelle
R¹, R² et R⁵ ont la définition indiquée ci-dessus,
qu'on transforme dans une autre étape par réaction avec l'acide chlorosulfonique en les composés de formule générale (V) 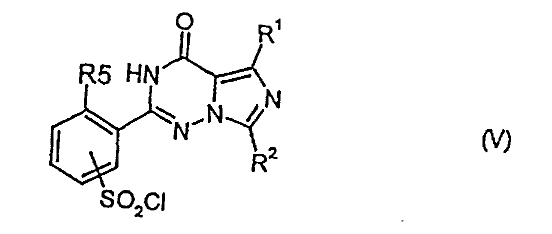 dans laquelle
R¹, R² et R⁵ ont la définition indiquée ci-dessus,
qu'on fait finalement réagir avec les amines correspondantes dans des solvants inertes.

7. Médicament contenant au moins un composé suivant l'une des revendications 1 à 4 ainsi que des agents de formulation acceptables du point de vue pharmacologique.

8. Médicament suivant la revendication 7, destiné au traitement de maladies cardiovasculaires, cérébrovasculaires et/ou de maladies des voies urogénitales.

9. Médicament suivant la revendication 8, destiné au traitement de troubles de l'érection.

10. Utilisation de composés suivant l'une des revendications 1 à 4 pour la préparation des médicaments.

11. Utilisation suivant la revendication 10, dans laquelle le médicament agit contre les troubles de l'érection.
